# EUROPÄISCHE PATENTANMELDUNG

(11) **EP 1 944 370 A1**
(43) Veröffentlichungstag der Anmeldung: **16.07.2008**
(21) Anmeldenummer: 08004552.9
(22) Anmeldetag: 06.08.1999
(51) Int. Cl.: C12N 15/12, C07K 14/705, C12Q 1/68, G01N 33/50, C07K 16/28

(54) **Neuer spannungsabhängiger Kaliumkanal und seine Verwendung zur Entwicklung von Therapeutika**

(30) Priorität: 06.08.1998 DE 19841413
(62) Teilanmeldung aus: 99941625.8
(71) Anmelder: Evotec AG, 22525 Hamburg (DE)
(72) Erfinder: Netzer, Rainer, 22549 Hamburg (DE); Pongs, Olaf, 20249 Hamburg (DE)
(74) Vertreter: UEXKÜLL & STOLBERG

(57) **Zusammenfassung**

Gegenstand der Erfindung ist ein neues spannungsabhängiges Kaliumkanalprotein, Kv6.2. (SEQ ID NO: 1) Das Kv6.2 Gen wird präferentiell im Myocard und im Hippocampus exprimiert. In Verbindung mit der Untereinheit Kv2.1 werden neuartige funktionelle heteromultimere Kaliumkanäle ausgebildet, die eine hohe Affinität zu Propafenon besitzen. Erfindungsgemäß werden diese neuartigen Kaliumkanäle für Testsysteme verwendet, die geeignet sind, Stoffe zu identifizieren, die Kv2.1/Kv6.2 Kanäle modulieren, öffnen bzw. schließen können und die damit als Therapeutika eingesetzt werden können.

## Beschreibung

Gegenstand der Erfindung ist ein neues spannungsabhängiges Kaliumkanalprotein, Kv6.2. (SEQ ID NO: 2 und 4). Ferner werden im Rahmen der vorliegenden Erfindung erstmals heterologe Kaliumkanäle zur Verfügung gestellt, die das Kaliumkanalprotein sowie weitere Kaliumkanaluntereinheiten, wie z.B. das Kv2.1 Protein enthalten. Ferner sind erfindungsgemäß Vektoren eingeschlossen, die die Kaliumkanaluntereinheit Kv6.2 enthalten, sowie diese Vektoren enthaltende Wirtszellen, die die Kaliumkanaluntereinheit bzw. die Kaliumkanäle exprimieren. Gegenstand der vorliegenden Erfindung sind ferner gegen die Kaliumkanaluntereinheit gerichtete Antikörper. Ferner wird erstmals ein Verfahren zum Identifizieren von Substanzen zur Verfügung gestellt, die Kaliumkanäle öffnen, schließen, aktivieren, inaktivieren oder in ihren biophysikalischen Eigenschaften verändern können. Gemäß einer besonderen Ausführungsform der Erfindung dient das Verfahren zum Identifizieren bzw. Auffinden von Antiarrhytmika.

Die Membranen von Säugetierzellen sind für die strukturelle Integrität und die Aktivität von Zellen und Gewebe von großer Bedeutung. Eine Vielzahl von Stoffwechselprozessen wird von Membran-durchspannenden Ionenkanälen gesteuert. In der Vergangenheit konnten verschiedene Ionenkanäle identifiziert werden, durch die Kalzium, Natrium und /oder Kalium die Zellmembran passieren können.

Die Aktivität von Kaliumkanälen kann entweder durch intrazelluläre Signalstoffe wie cAMP oder durch Potentialdifferenzen an der Zellmembran reguliert werden. Diese Potentialdifferenzen oder Spannungen kommen durch unterschiedliche Ionenkonzentrationen innerhalb und außerhalb der Zelle zustande.

Spannungsabhängige Kaliumkanäle sind abhängig je nach des entlang der Zellmembran vorliegenden Potentials geöffnet oder geschlossen. Es sind verschiedene Klassen von spannungsabhängigen Kaliumkanälen bekannt, deren Aufbau in der Regel ähnlich ist. Grundsätzlich bestehen sie aus vier homologen α-Untereinheiten und vier ß-Untereinheiten. Die ß-Untereinheiten sind für die Regulation der Aktivität des Kanales wichtig, während die α-Untereinheiten den eigentlichen funktionellen Kaliumkanal bilden (O. Pongs, Biospektrum 3 (1997) 21-26). Die α-Untereinheiten gehören zu einer gemeinsamen Gensuperfamilie. Sie besitzen eine vergleichbare zweidimensionale Struktur, aus der eine für Kaliumkanäle typische Membrantopologie hervorgeht (O. Pongs, Physiol. Rev. 72 (1992) 69-88.; L.Y. Jan et al., Nature 371 (1994) 119-122; K.G. Chandy et al., in Handbook of Receptors and Channels ed. R.A. North, Boca Raton 1 (1994) 1-71). Jede α-Untereinheit besitzt sechs hydrophobe Membran-durchspannende Segmente S1-S6 und zwischen S5 und S6 die sogenannte P-Region, die von der extrazellulären Seite in die Membran eintaucht. Die P-Region hat einen entscheidenden Anteil an der Ausbildung der Kaliumkanalpore. Das S4-Segment enthält mehrere Aminosäuren mit positiven Ladungen, die wahrscheinlich für die Spannungsempfindlichkeit des Kanals einen wesentlichen Beitrag liefern.

Die Familie der spannungsabhängigen Kaliumkanaluntereinheiten läßt sich in mehrere Unterfamilien unterteilen, von denen die Kv1- bis Kv4-Familien gut charakterisiert sind (W. Stühmer et al., EMBO J. 8 (1989) 3235-3244; B. Albrecht et al., Receptor and Channel 1 (1993) 99-100; J. Rettig et al., EMBO J. 11 (1992) 2473-2486; Serodio et al., J. Neurophysiol. 75 (1996) 2174-2179). Innerhalb der Unterfamilien liegt die Sequenzidentität der einzelnen α-Untereinheiten untereinander auf der Ebene der Aminosäuren bei ≥60%. Die bisher klonierten α-Untereinheiten der Familien Kv1 bis Kv4 exprimieren funktionelle Kaliumkanäle in heterologen Expressionssystemen, d.h. nach Injektion von DNA und mRNA in Xenopus Oozyten bzw. in Gewebekulturzellen (Chinese Hamster Ovary (CHO) Zellen, Human Epithelial Kidney (HEK) 293 Zellen) oder nach Transfektion von Gewebekulturzellen mit für α-Untereinheiten kodierender DNA in geeigneten Expressionsvektoren wie pcDNA3 (s.u.).

Zusätzlich zu α-Untereinheiten von Kv1 bis Kv4 sind noch weitere potentielle Kv α-Untereinheiten bekannt (M.A. Post et al., FEBS 399 (1996) 177-182; J.P. Hugnot et al., EMBO 15 (1996) 3322-3331; A. Castellano et al., J. Neurosci. 17 (1997) 4652-4661; J.A. Drewe et al., J. Neurosci. 12 (1992) 538-548, die zu Kv1 bis Kv4 bezogen auf die Aminosäuren eine Sequenzidentität von <60% zeigen. Diese Kanäle wurden als Kv5.1, Kv6.1, Kv7.1, Kv8.1 bezeichnet. Hauptmerkmal dieser α-Untereinheiten ist, daß sie zwar für Kaliumkanal α-Untereinheiten typische Sequenzmerkmale enthalten, aber als Homomultimere keine funktionellen Kanäle in heterologen Expressionsystemen ausbilden. Es ist aber möglich, daß diese α-Untereinheiten zusammen mit α-Untereinheiten der Kv2 Familie Heteromultimere ausbilden, die funktionell exprimierbar sind, d.h. funktionelle Kaliumkanäle bilden (M.A. Post et *al.* (a.a.O.); J.P. Hugnot et *al.* (a.a.O.); A. Castellano et *al.* (a.a.O.).

Spannungsabhängige Kaliumkanäle können vielfältige physiologische Aufgaben übernehmen, die von der Regulation des Membranruhepotentials bis hin zur Regulation der Exozytose und Zellproliferation reichen. In erregbaren Zellen haben spannungsabhängige Kaliumkanäle eine wichtige Bedeutung für die Repolarisation der Aktionspotentiale und die Regulation des Schwellenwertes, von dem aus ein Aktionspotential ausgelöst werden kann. Insofern steuert die Aktivität von Kaliumkanälen sowohl die Dauer und Verlaufsform des Aktionspotentials als auch die Aktionspotentialauslösungsfrequenz. Dies gilt auch für die rhythmische Erzeugung von Aktionspotentialen im Herzmuskelgewebe, dem Myocard (R.E. Ten Eick et al., FASEB J. 6 (1992) 2568-2580).

Mehrere distinkte Kaliumkanaltypen sind an der Generierung und Repolarisierung der Aktionspotentiale im Myocard beteiligt. Die von diesen Kanälen generierten Ströme werden als I_{TO}, I_{KR} und I_{SK} bezeichnet. I_{TO} ist ein schnell aktivierender transienter Kaliumauswärtsstrom, I_{KR} ist ein schnell aktivierender, nicht inaktivierender Kaliumauswärtsstrom, I_{SK} ist ein langsam aktivierender Kaliumauswärtsstrom. Diese Ströme wurden an dissoziierten, in Kultur gehaltenen Myocardzellen gemessen (R.C. Kass und L.C. Freeman, Trends Cardiovasc. Med. 3 (1993) 149-159; D.M. Barry und J.M. Nerbonne, Ann. Rev. Physiol. 58 (1996) 363-394). Die Analyse von erblichen Herzrhythmusstörungen, die zu einem langen QT-Syndrom, d.h. einer verzögerten Repolarisierung des kardiakalen Aktionspotentials, führen, hat gezeigt, daß der I_{SK}-Strom im wesentlichen durch KvLQT1/Kv-Kanäle vermittelt wird (M.C. Sanguinetti et al., Nature 384 (1996) 80-83; J. Berhamin et al., Nature 384 (1996) 78-80). HERG/Kv-Kanäle vermitteln Ströme, die zur Nachhyperpolarisierung und damit zur Stabilisierung des Schwellenwertes beitragen (P.L. Smith et al., Nature 379 (1996) 833-836). Pharmakologisch ist es möglich, HERG-Kanäle relativ spezifisch durch Pharmaka wie E-4031 zu blockieren (P.S. Spector et al., Cir. Res. 78 (1996) 499-503). Vermutlich sind Kanäle des Typus Kv1.5 und Kv4.3 sowie die hier beschriebenen Kv2.1/Kv6.2 Kanäle an der Ausbildung des I_{TO} und I_{KR} beteiligt (vgl. R.C. Kass und L.C. Freeman, Trends Cardiovasc. Med. 3 (1993) 149-159; D.M. Barry und J.M. Nerbonne, Ann. Rev. Physiol. 58 (1996) 363-394). Es ist noch nicht bekannt, welche und wieviele Kaliumkanäle insgesamt an der Repolarisierung des kardiakalen Aktionspotentials beteiligt sind.

Herzrhythmusstörungen werden gegenwärtig häufig mit Ionenkanalblockern behandelt. Die Wirkungsweise dieser Blocker läßt sich dahingehend klassifizieren, ob sie die Depolarisierungsgeschwindigkeit (Ansstieg) des kardiakalen Aktionspotentials verzögern (z.B. Flecainid, Phenytoin) bzw. die Dauer des kardiakalen Aktionspotentials verlängern (z.B. Sotalol, Aminodaron, Chinidin, Disopyramid) oder aber die Dauer des kardiakalen Aktionspotentials verkürzen (z.B. Lidocain, Mexiletin). Ein wichtiges Präparat ist die Substanz Propafenon (Merck-Index XI 7806), die zu den Antiarrhytmika der Klasse 1 c gezählt wird (H. Honjo et al. Br. J. Pharmacol. 97 (1989) 731-738). Propafenon wird bei symptomatischen und behandlungsbedürftigen tachykarden supraventrikulären Herzrhythmusstörungen, wie z. B. AV junktionale Tachykardien, supraventrikuläre Tachykardien bei WPW-Syndrom oder paroxysmales Vorhofflimmern und schwerwiegend symptomatische ventrikuläre tachykarde Herzrhythmusstörungen eingesetzt (J. Braun und R. Preuss. Klinikleitfaden Intensivmedizin, 2. Auflage, Jung Johann Verlagsgesellschaft, Neckarsulm/Stuttgart (1992)). Die Verabreichung von Propafenon führt, wie in einigen experimentellen Arbeiten beschrieben, nach einem Verschluß der Herzgefäße zu einer verbesserten metabolischen und funktionellen Erholung des Herzens (J.X. Liu et al. Eur. J. Pharmacol. 250/1 (1993) 361-369).

Für den Wirkungsmechanismus von Propafenon wird eine Blockade spannungsabhängiger Natrium-Kanäle angenommen. Propafenon blockiert zusätzlich den L-type Kalzium-Kanal, eine Reihe von Kalium-Kanälen und β-adrenerge Rezeptoren (A.O. Grant J. Cardiovasc. Elektrophysiol. 7 (1996) 353-364). Bei relativ hohen Konzentrationen sind Interaktionen mit den einzelnen Ionenkanälen und β-adrenergen Rezeptoren diskutiert worden (J.C. Hancox und J.S. Mitcheson. Br. J. Pharmacol. 121 (1997) 7-14; B. Koller und M.R. Franz. J. Cardiovasc. Pharmacol. 24 (1994) 753-760; G. Malfatta et al. Eur. Heart J. 14 (1993) 1253-1257). Ein Kanal, für den Propafenon eine hohe Bindungsaffinität hat, ist im Stand der Technik jedoch nicht bekannt.

Propafenon weist jedoch den Nachteil auf, daß beim Patienten vorübergehend Kopfschmerzen, Schwindel, Augenflimmern oder Magen-Darm-Störungen auftreten können (J. Braun und R. Preuss. Klinikleitfaden Intensivmedizin, 2. Auflage, Jung Johann Verlagsgesellschaft, Neckarsulm/Stuttgart (1992). Bei älteren Patienten kommen häufig hypotone Kreislaufstörungen vor. Bei stark vorgeschädigtem Myokard können unerwünschte Beeinträchtigungen der Erregungsleitung im HIS-Purkinje-System und der Myokardkontraktilität auftreten (P. Vigreux et al. Therapie 50 (1995) 413-418; P.J. Podrid und J.L. Anderson. Am. J. Cardiol. 15 (1996) 430-434; E. Aliot und I. Denjoy. Am. J. Cardiol. 77 (1996) 66A-71A).

Aufgrund der Nebenwirkungen von Propafenon und anderen im Stand der Technik bekannten Antiarrhythmika wird ständig nach neuen Wirkstoffen gesucht. Das gezielte Screening nach neuen Antiarrhythmika erfolgt in der Pharma-Industrie bislang in der Regel mit Hilfe einer Langendorf-Apparatur. Dabei wird die Funktion eines isolierten Kaninchen- oder Mäuseherzens unter entweder einem konstanten Druck oder einem konstanten Fluß gemessen (A. von Bethmann et al. Am. J. Respir. Crit. Care Med. 153 (1996) A529).

Aufgabe der vorliegenden Erfindung ist es daher, ein neues Testsystem (Assay) zur Verfügung zu stellen, das geeignet ist, Stoffe auf ihre Eignung als Antiarrhythmika zu testen, d.h. mit dem getestet werden kann, ob sich Wirkstoffe als Antiarrhythmika eignen. Insbesondere soll der Assay verwendet werden können, um auf einfache Weise gezielt Wirkstoffe daraufhin testen, ob sie die Modulation von I_{KR}-Strömen ermöglichen. Mit dem Testsystem sollen Pharmaka somit auf ihre Wirkung gegenüber I_{KR}-Strömen überprüft werden können. Insbesondere ist es Aufgabe der vorliegenden Erfindung, ein Testsystem zur Verfügung zu stellen, das den bislang notwendigen Einsatz der Langendorf-Apparatur überflüssig macht oder zumindest stark einschränkt.

Erfindungsgemäß wird die Aufgabe durch Wirtszellen gelöst, die den spannungsabhängigen Kaliumkanal Kv2.1/Kv6.2 exprimieren.

Im Rahmen der vorliegenden Erfindung wird überraschenderweise eine neue Untereinheit eines Kaliumkanalproteins, Kv6.2, zur Verfügung gestellt, die in Verbindung mit Kv2.1-Untereinheiten nicht-inaktivierende Kaliumauswärtsströme vermittelt, die aufgrund ihrer Eigenschaften zu I_{KR}-Strömen beitragen können. Im Rahmen der vorliegenden Erfindung zeigte es sich, daß die erfindungsgemäßen Kv2.1/Kv6.2-Kanäle hochsensitiv gegen das Klasse IC Herzantiarrhythmikum Propafenon sind. Die erfindungsgemäßen Kaliumkanäle eignen sich in besonderer Weise zur gezielten Identifizierung und Entwicklung von Wirkstoffen zur Behandlung von Erkrankungen des Herzkreislaufsystems und des Nervensystems in Mensch und Tier, insbesondere von Antiarrhytmika.

Das erfindungsgemäße humane Kaliumkanalprotein weist die in SEQ ID NO: 2 gezeigte Aminosäuresequenz auf.

Das erfindungsgemäße murine Kaliumkanalprotein weist die in SEQ ID NO: 4 gezeigte Aminosäuresequenz auf.

Erfindungsgemäß eingeschlossen sind auch Homologe, d.h., im Myocard exprimierte Kaliumkanalproteine des Kv6.2 Typs mit mindestens 60% Sequenzidentität, sowie Derivate oder Fragmente der Kaliumkanalproteine, die die gleiche elektrophysiologische, pharmakologische und biologische Wirksamkeit und/oder Immunogenität aufweisen.

Überraschenderweise wurde festgestellt, daß die bislang unbekannte Kaliumkanaluntereinheit Kv6.2 im Vorhof des Herzens von Säugern prominent exprimiert wird (siehe Beispiel 7). In Northern Blots von mRNA extrahiert aus verschiedenen humanen Geweben wurde Kv6.2 mRNA zusätzlich noch in Leber, Skelettmuskel, Niere, Pankreas und in sehr geringen Mengen in Gehirn, Lunge und Placenta gefunden.

Das erfindungsgemäße Kaliumkanalprotein sowie Homologe, Derivate oder Fragmente desselben mit gleicher elektrophysiologischer, pharmakologischer und/oder biologischer Wirksamkeit und/oder Immunogenität sind auf verschiedenen, dem Fachmann bekannten Wegen erhältlich. Zum einen können das Kaliumkanalprotein oder Homologe, Derivate oder Fragmente desselben mittels chemischer Synthese hergestellt werden. Desweiteren können Antikörper gegen Fragmente des Polypeptids nach dem Fachmann bekannten Methoden hergestellt werden (E. Harlow und D. Lane, Antibodies: A Laboratory Manual (1988) Cold Spring Harbor Laboratory, Cold Spring Harbor, NY.). Mittels dieser Antikörper kann dann das erfindungsgemäße Kaliumkanalprotein oder Derivate und Fragmente desselben aus Zellen isoliert werden, die es exprimieren. Dabei kann es sich um Zellen handeln, die natürlicherweise das Kaliumkanalprotein exprimieren, es ist aber ebenso denkbar, daß Zellen verwendet werden, in die für das erfindungsgemäße Kaliumkanalprotein kodierende Nukleinsäuremoleküle eingeführt werden und die das Protein anschließend unter geeigneten Bedingungen exprimieren.

Gegenstand der Erfindung ist ferner ein Kaliumkanal, der dadurch gekennzeichnet ist, daß er mindestens die Kaliumkanaluntereinheit Kv6.2 enthält. Erfindungsgemäß kann der Kaliumkanal neben der Untereinheit Kv6.2 auch andere Kaliumkanaluntereinheiten enthalten. Dabei kommen besonders die Untereinheiten Kv2. 1, Kv2. 2 und Kv2.3 in Frage. Besonders bevorzugt enthält er zusätzlich die Raliumkanaluntereinheit Kv2.1. Die spezifischen Eigenschaften des Kaliumkanals hängen von den Kaliumkanaluntereinheiten ab, die er neben Kv6.2 enthält. Enthält er neben Kv6.2 die Kaliumkanaluntereinheit Kv2.1, so handelt es sich um einen spannungsabhängigen Kaliumkanal, der bei Depolarisierung der Membran Auswärtsströme vermittelt.

Gegenstand der Erfindung sind ferner Nukleinsäuremoleküle, die dadurch gekennzeichnet sind, daß sie für die erfindungsgemäßen Kaliumkanalproteine und Kaliumkanäle, deren Homologe, Derivate und/oder Fragmente mit gleicher elektrophysiologischer, pharmakologischer und/oder biologischer Wirksamkeit und Immunogenität kodieren. Besonders bevorzugt können diese erfindungsgemäßen Nukleinsäuremoleküle ausgewählt werden aus
a) der in SEQ ID NO: 1 angegebenen Nukleotidsequenz,
b) syngenen oder komplementären Sequenzen der Sequenzen gemäß a), soweit sie für Proteine und Polypeptide mit gleicher elektrobiologischer, pharmakologischer und/oder biologischer Wirksamkeit und/oder Immunogenität kodieren, und
c) allelischen Varianten und Fragmenten der Sequenzen gemäß a) und b).

Bestandteil der Erfindung ist ferner ein Vektor, der dadurch gekennzeichnet ist, daß er eines oder mehrere der oben genannten Nukleinsäuremoleküle enthält. Geeignete Vektoren sind pBluescript KS⁺und pBluescript KS⁻ (Stratagene, La Jolla, CA, US), sind aber nicht auf diese beschränkt. Gemäß einer bevorzugten Ausführungsform der Erfindung ist der Vektor ein Expressionsvektor. Ein geigneter Expressionsvektor ist pcDNA3 (Invitrogene, Carlsbad, CA, US), die Erfindung ist aber nicht auf diesen beschränkt. Die erfindungsgemäßen Nukleinsäuremoleküle können in diese Vektoren nach allgemeinen bekannten Methoden kloniert werden (T. Maniatis et al., Molecular Cloning: A Laboratory Manual (1982) Cold Spring Harbor Laboratory, Cold Spring Harbor, NY, US). Erfindungsgemäß enthalten die Expressionsvektoren Kontrollelemente für Transkription, Transkriptionsstart, Transkriptionsende, mRNA-Prozessierung und Translation, die in den erfindungsgemäß verwendeten Expressionssystemen in aktiver Form vorliegen.

Bevorzugt enthalten die erfindungsgemäßen Vektoren Sequenzen, die die Replikation der erfindungsgemäßen Nukleinsäuremoleküle erleichtern. Besonders bevorzugt enthalten sie ferner Sequenzen, die die Integration der Nukleinsäuremoleküle in das Genom einer Wirtszelle erleichtern.

Gegenstand der Erfindung sind ferner Wirtszellen, die mit den erfindungsgemäßen Vektoren transformiert sind. Besonders bevorzugt sind diese Wirtszellen CHO-Zellen oder Xenopus Oozyten. Als Wirtszellen kommen aber auch andere Eukaryontenzellen aus der Gruppe bestehend aus COS, HEK 293, NIH-3T3 in Frage, sind aber nicht auf diese beschränkt. Von Bedeutung ist, daß die Promotor- und/oder Enhancersequenzen auf die mit den Vektoren transformierten Wirtszellen abgestimmt sind. Dadurch kann eine erhöhte Expression der erfindungsgemäßen Polypeptide sichergestellt werden.

Ferner ist eine Wirtszelle Gegenstand dieser Erfindung, die dadurch gekennzeichnet ist, daß sie neben den erfindungsgemäßen Vektoren zusätzlich mit einem weiteren Vektor transformiert ist, der eine Nukleinsäuresequenz enthält, die für eine andere Kaliumkanaluntereinheit kodiert. Besonders bevorzugt kodiert diese Nukleinsäuresequenz für die Kaliumkanaluntereinheit Kv2.1 (B. Albrecht et al. Receptor and Channnel 1 (1993) 99-100). Sie kann aber auch für andere Kaliumkanaluntereinheiten wie Kv2.2 und Kv2.3 kodieren.

Gegenstand der vorliegenden Erfindung ist ferner eine Wirtszelle, die einen funktionellen Kaliumkanal exprimiert, der die Kaliumkanaluntereinheit Kv6.2 enthält. Die erfindungsgemäße Wirtszelle exprimiert den funktionellen Kaliumkanal vorzugsweise auf ihrer Oberfläche, es ist aber ebenso möglich, daß der funktionelle Kaliumkanal in intrazellulären Membranen exprimiert ist.

Erfindungsgemäß eingeschlossen ist ferner ein Verfahren zum Identifizieren und Testen von Substanzen, die geeignet sind, die von den erfindungsgemäßen Wirtszellen exprimierten Kaliumkanäle zu öffnen, zu schließen, zu aktivieren, zu inaktivieren und/oder in ihren biophysikalischen Eigenschaften zu verändern, bei dem man
a) an den erfindungsgemäßen Wirtszellen den Kaliumauswärtsstrom mißt,
b) die Wirtszellen mit einer zu untersuchenden Substanz in Kontakt bringt und
c) an den Wirtszellen erneut den Kaliumauswärtsstrom mißt,
wobei der Unterschied zwischen den Kaliumauswärtsströmen vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die hinzuzusetzende Konzentration der Substanz ist, um eine Änderung der Kaliumauswärtsströme zu erreichen.

Besonders bevorzugt exprimieren die verwendeten Wirtszellen den erfindungsgemäßen Kaliumkanal auf ihrer Oberfläche. Auf diese Weise können die Substanzen dadurch identifiziert bzw. getestet werden, daß man den Ausstrom von Ionen aus den Zellen durch den erfindungsgemäßen Kaliumkanal mißt. Das Ausströmen von Ionen wird bevorzugt mit der 'patch-clamp'-Methode (vgl. z.B. O.P. Hamill et al., Pflügers Arch. (1981) 85-100) durch Anlegen depolarisierender Testpotentiale bestimmt.

Im Rahmen der vorliegenden Erfindung ist ferner eingeschlossen, die erfindungsgemäßen Wirtszellen nach dem Fachmann gut bekannten Methoden mit ⁸⁶Rb-Ionen zu beladen, die durch Kaliumkanäle so gut wie Kaliumionen permeieren können. Die beladenen Zellen können in Gegenwart von zu testenden Substanzen kultiviert werden. Danach kann der Einfluß der Substanzen auf den ⁸⁶Rb-Auswärtsstrom der mit ⁸⁶Rb beladenen Zellen mit dem Fachmann bekannten Methoden gemessen werden (R.S. Rogowski et al. Mol. Pharmacol. 50 (1996) 1167-1177).

Erfindungsgemäß wird eine Substanz als eine öffnende Substanz bezeichnet, wenn nach Zugabe der Substanz bei einem Membranpotential, bei dem ohne Zugabe der Substanz keine Kaliumauswärtsströme fließen, Kaliumauswärtsstöme fließen.

Erfindungsgemäß wird eine Substanz als eine aktivierende Substanz bezeichnet, wenn nach Zugabe der Substanz ein bereits vorhandener Kaliumauswärtsstrom verstärkt wird.

Erfindungsgemäß wird eine Substanz als eine schließende Substanz bezeichnet, wenn nach Zugabe der Substanz bei einem Membranpotential, bei dem ohne Zugabe der Substanz Kaliumauswärtsströme fließen, keine Kaliumauswärtsströme fließen.

Erfindungsgemäß wird eine Substanz als eine inaktivierende Substanz bezeichnet, wenn nach Zugabe der Substanz ein bereits vorhandener Kaliumauswärtsstrom vermindert wird, ohne daß der Kaliumauswärtsstrom vollständig zum Erliegen kommt.

Erfindungsgemäß wird eine Substanz als eine verändernde Substanz bezeichnet, wenn durch Zugabe der Substanz biophysikalische Eigenschaften des Kaliumkanals wie Spannungsabhängigkeit, Leitfähigkeit, Aktivierungszeitkonstanten, Inaktivierungszeitkonstanten, Schaltverhalten, Offenzeiten oder Geschlossenzeiten verändert werden.

Änderungen in der Spannungsabhängigkeit der Aktivierung führen erwartungsgemäß bei Testpotentialen, die Kaliumauswärtsströme hervorrufen, zu einer Stromzunahme oder Stromabnahme. Leitfähigkeitsänderungen führen ebenfalls zu einer Zu- oder Abnahme der Kaliumauswärtsströme. Änderungen der Aktivierungszeitkonstanten führen zu einer Verlangsamung oder Beschleunigung der Aktivierung von Kaliumauswärtsströmen. Änderungen der Inaktivierungszeitkonstanten sowie des Schaltverhaltens können während eines Testpulses zu einer Zunahme oder Abnahme der Auswärtsströme führen. Das gleiche gilt, wenn die Offenzeiten oder Geschlossenzeiten der zu messenden Kaliumkanäle verändert werden (B. Hille, Ionic Channels of Excitable Membranes, 2. Ausgabe (1993), Sinauer Associates Inc., Sunderland, Massachusetts, USA).

Erfindungsgemäß wird eine Substanz auch dann als eine verändernde Substanz bezeichnet, wenn durch Zugabe der Substanz die Zelloberflächenexpression des Kaliumkanals verändert wird. Eine Veränderung der Zelloberflächenexpression führt zu einer Zunahme bzw. Abnahme der zu messenden Kaliumauswärtsströme.

Gegenstand der Erfindung ist ferner ein Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, bei dem man
a) an den erfindungsgemäßen Wirtszellen das Membranpotential mißt,
b) die Wirtszellen mit einer Substanz in Kontakt bringt und
c) an den Wirtszellen erneut das Membranpotential mißt,
wobei der Unterschied zwischen dem Membranpotential vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die einzusetzende Konzentration der Substanz ist, um eine Änderung des Membranpotentials zu erreichen.

Gegenstand der Erfindung ist ferner ein Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, bei dem man
a) in den erfindungsgemäßen Wirtszellen das Membranpotential und den Kaliumauswärtsstrom mißt,
b) die Wirtszellen mit einer Substanz in Kontakt bringt und
c) das Membranpotential und den Kaliumauswärtsstrom erneut mißt,
wobei die Unterschiede zwischen dem Membranpotential und dem Kaliumauswärtsstrom vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die einzusetzende Konzentration der Substanz ist, um eine Änderung des Membranpotentials sowie des Kaliumauswärtsstromes zu erreichen.

Besonders bevorzugt sind die in den erfindungsgemäßen Verfahren verwendeten Wirtszellen Xenopus Oozyten. Ferner sind erfindungsgemäß CHO Zellen sowie andere Gewebekulturzellen wie COS-Zellen und HEK 293 Zellen bevorzugt, die Auswahl ist aber nicht auf diese beschränkt, solange in den verwendeten Wirtszellen ein funktioneller Kaliumkanal erhalten werden kann.

Überraschenderweise hat es sich im Rahmen der vorliegenden Erfindung gezeigt, daß funktionelle Kaliumkanäle, welche die Kaliumkanaluntereinheiten Kv6.2 und Kv2.1 enthalten, einen hochaffinen Rezeptor für Propafenon darstellen. Daher eignet sich das erfindungsgemäße Verfahren unter Verwendung von Wirtszellen, die den Kv2.1/Kv6.2-Kaliumkanal exprimieren, zum Auffinden und Testen von neuen oder bekannten Substanzen und Wirkstoffen, die einen Effekt auf den Herzrhythmus haben. Besonders bevorzugt eignet sich das erfindungsgemäße Verfahren zum Auffinden und Testen von Klasse IC Antiarrhythmika.

Somit ist im Rahmen der vorliegenden Erfindung die Identifizierung von Substanzen möglich, die dazu geeignet sind, die erfindungsgemäßen Kaliumkanäle zu öffnen, zu schließen, zu aktivieren, zu inaktivieren oder in ihren biopysikalischen Eigenschaften zu verändern. Aufgrund der spezifischen Lokalisation und Funktion dieser Kanäle können deren Modulatoren (aktivierend oder inaktivierend) in unterschiedlichen cardiovaskulären und neuronalen Bereichen eine Behandlung ermöglichen.

Im cardiovaskulären Bereich sind außer dem Herzryhthmus die Kontraktionskraft und die Herzdurchblutung von Bedeutung. Modulatoren der erfindungsgemäßen Kaliumkanäle können somit potentiell in der Therapie von Arrhythmien oder Bluthochdruck sowie bei der Cardioprotektion eingesetzt werden.

Im neuronalen Bereich spielen Kaliumkanäle eine entscheidende Rolle in der Regulation der Aktivität von Neuronen. Modulatoren dieser Kaliumkanäle können potentiell Lern- und Gedächtnisfunktionen beeinflussen und beispielsweise bei neurodegenerativen Erkrankungen (z.B. Epilepsien, Ischämien, Schlaganfall, Morbus Parkinson und Alzheimer) therapeutisch eingesetzt werden.

Ferner werden im Rahmen der vorliegenden Erfindung Antikörper zur Verfügung gestellt, die an das isolierte erfindungsgemäße Kaliumkanalprotein oder an Derivate oder Fragmente desselben mit gleicher elektrobiologischer, pharmakologischer und/oder biologischer Wirksamkeit und/oder Immunogenität binden. Ferner werden Antikörper zur Verfügung gestellt, die an das erfindungsgemäße Kaliumkanalprotein oder an Derivate oder Fragmente desselben mit gleicher elektrophysiologischer, pharmakologischer und/oder biologischer Wirksamkeit und/oder Immunogenität binden,
wobei das Kaliumkanalprotein oder die Derivate oder Fragmente desselben mit gleicher elektrophysiologischer, pharmakologischer und/oder biologischer Wirksamkeit und/oder Immunogenität Bestandteil eines Kaliumkanales sind und somit eine andere dreidimensional Struktur aufweisen können als die isolierten erfindungsgemäßen Kaliumkanalproteine. Verfahren zur Herstellung von Antikörpern sind dem Fachmann allgemein bekannt (E. Harlow und D. Lane, a.a.O.). Die Antikörper sind erhältlich, indem man Tiere mit dem erfindungsgemäßen Kaliumkanalprotein oder Derivaten oder Fragmenten desselben mit gleicher elektrophysiologischer, pharmakologischer und/oder biologischer Wirksamkeit und/oder Immunogenität immunisiert. Polykonale Antikörper werden dann aus dem Serum der Tiere gewonnen, während monoklonale Antikörper aus dem Überstand von Hybridomzellen erhältlich sind. Hybridomzellen sind erhältlich, indem man Antikörper produzierende Zellen mit Tumorzellen fusioniert (E. Harlow and D. Lane, a.a.O.).

Erfindungsgemäß eingeschlossen sind auch Spezieshomologe des erfindungsgemäßen humanen Kaliumkanalproteins Kv6.2 sowie deren Derivate und/oder Fragmente mit gleicher Immunogenität. Die Spezieshomologe zeichnen sich dadurch aus, daß sie aus vom Menschen verschiedenen Spezies entstammen und eine Aminosäureidentität von mindestens 60 % zum erfindungsgemäßen humanen Kaliumkanalprotein aufweisen und - wie bereits oben beschrieben - zusammen mit anderen Kaliumkanaluntereinheiten Kaliumkanäle bilden, die vorzugsweise Klasse IC Antiarrhythmika binden.

Besonders bevorzugt stammt das Spezieshomologe aus der Maus und weist die in SEQ ID NO: 4 gezeigte Aminosäuresequenz auf.

Die Erfindung wird im folgenden durch Beispiele, Sequenzprotokolle und Figuren verdeutlicht.

### Kurze Beschreibung der Figuren

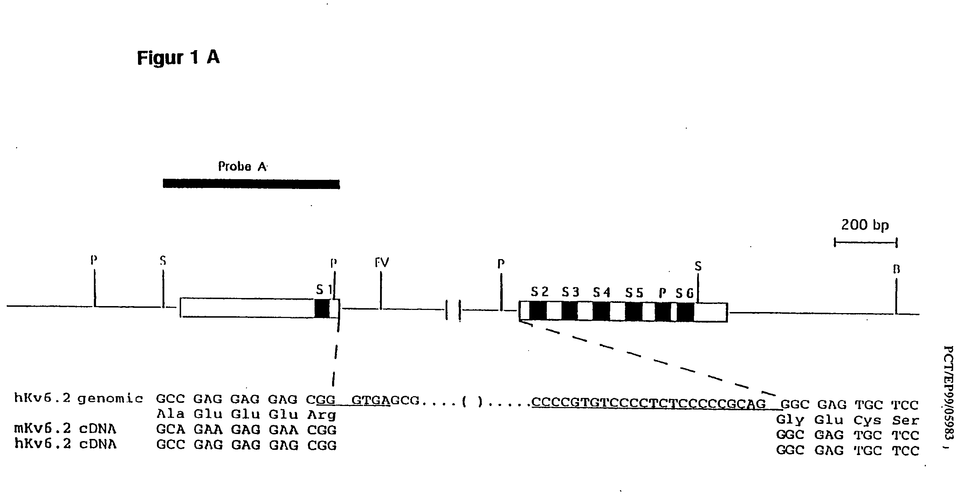

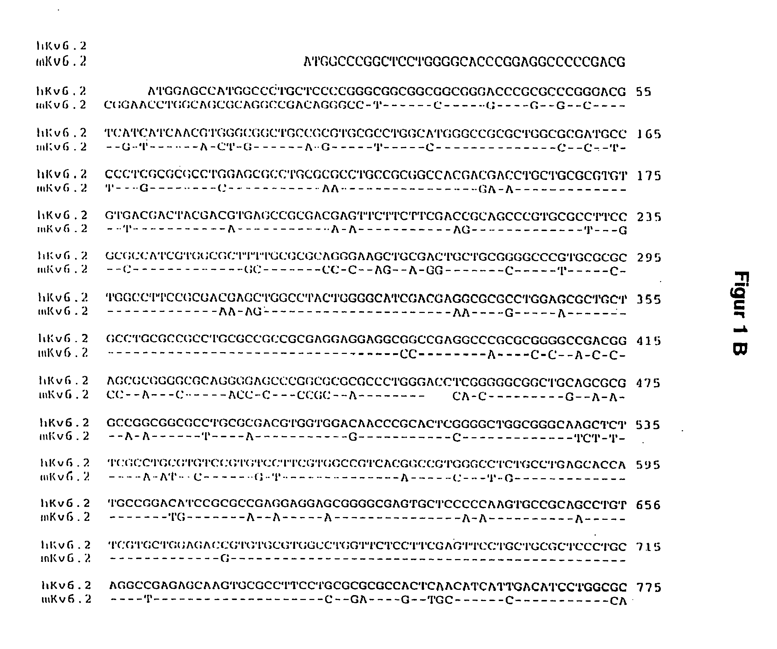

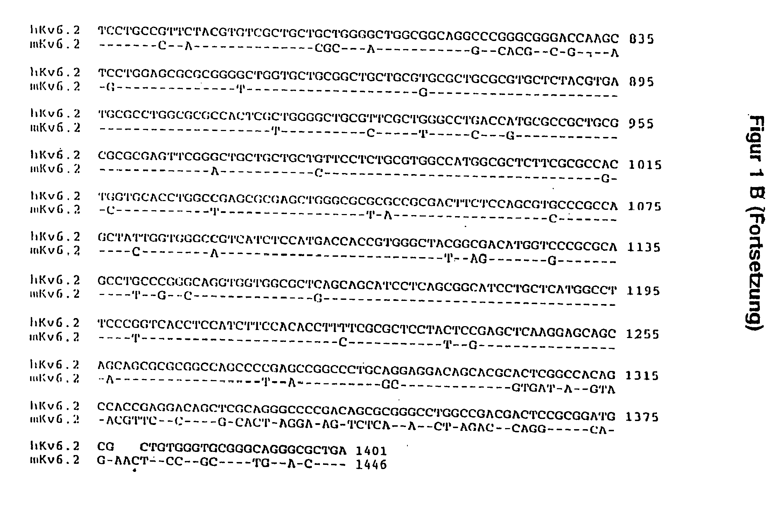

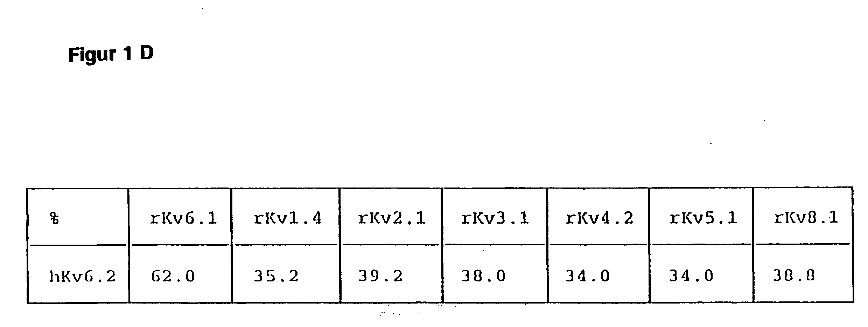

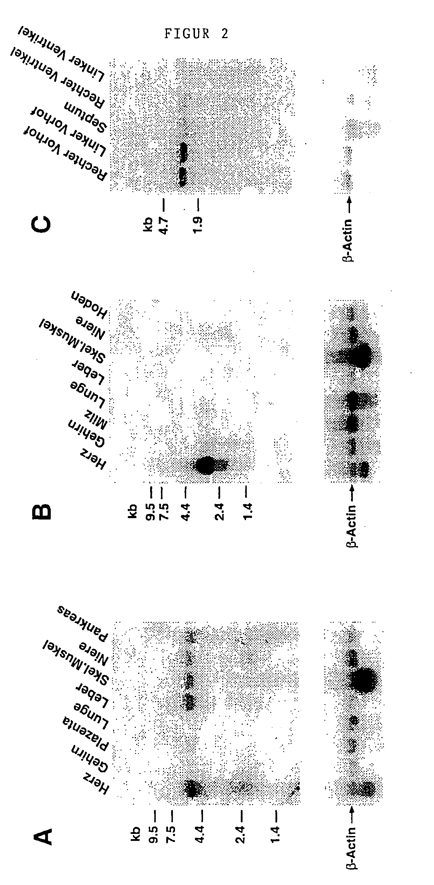

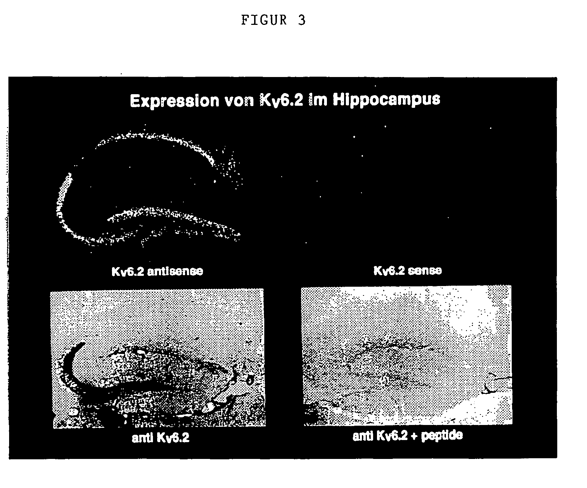

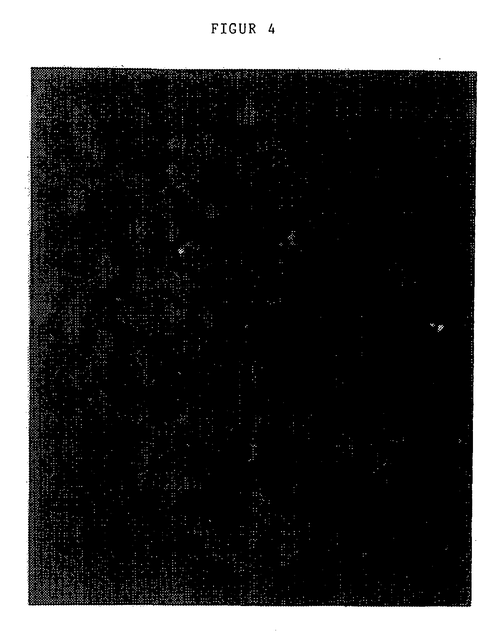

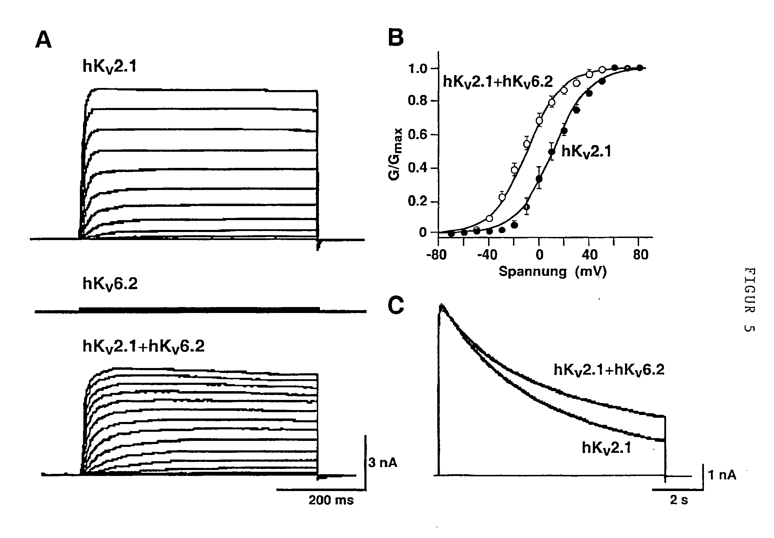

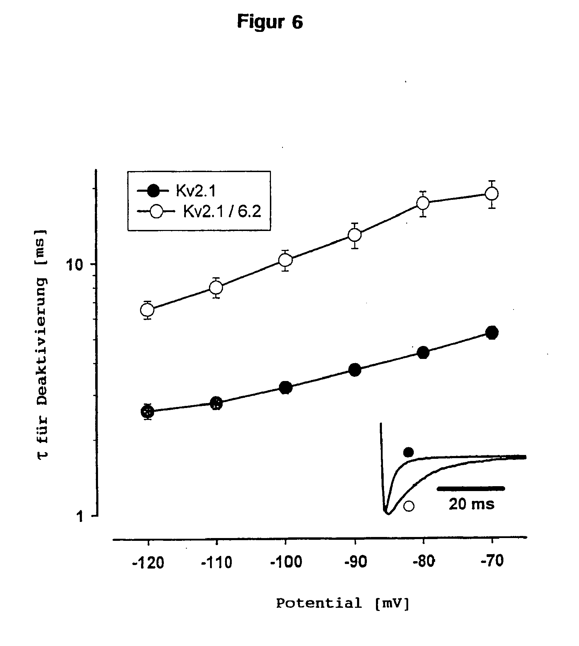

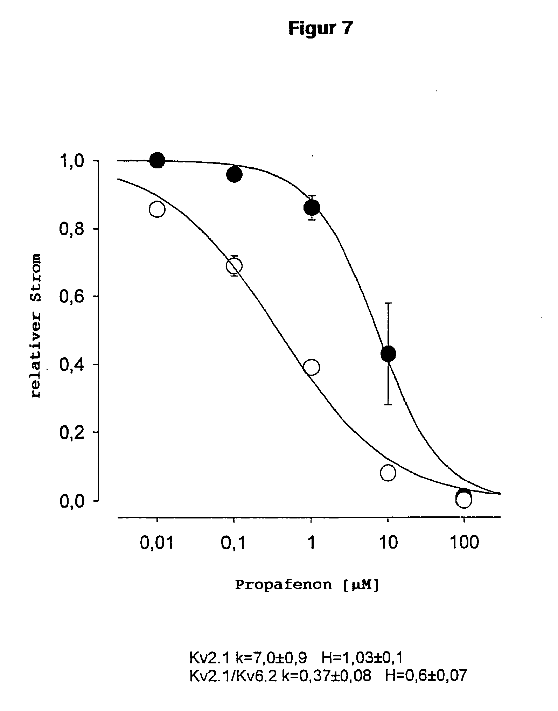

Figur 1
   A) Genomische Organisation des humanen Kv6.2 Gens
      Die kodierende Region ist schematisch durch zwei Rechtecke dargetellt. Die sieben schwarzen Balken zeigen die sechs hydrophoben, möglicherweise membranspannenden Segmente S1 bis S6 und das an der Porenbildung beteiligte Segment P an. Restriktionsschnittstellen sind wie folgt abgekürzt: EV-EcoRV, P-PstI, S-SacI, B-BamHI. Diese Restriktionsschnittstellen wurden durch einzelne bzw. doppelte Verdauungen und die Sequenzierung lokalisiert. Die Exon-Intron Übergänge (SEQ ID NO: 9 und 13) wurden durch Vergleich der genomischen Sequenz des humanen Kv6.2 mit der humanen Kv6.2 cDNA (hKv6.2 cDNA SEQ ID NO: 1) bzw. der Maus cDNA (mKv6.2 cDNA SEQ ID NO: 3) erhalten. Das humane genomische SacI/PstI-Fragment (Probe A) wurde als Hybridisierungssonde für die Northern-Analyse in Figur 2 verwendet. Die gestrichelten Linien verweisen auf die genomischen Sequenzen der Exon-Intron Übergänge. Intronsequenzen sind unterstrichen. Unterhalb der hKv6.2 Sequenz ist die abgeleitete Teilproteinsequenz angegeben. mKv6.2 cDNA und hKv6.2 cDNA zeigt die cDNA-Sequenzen im Bereich der Exon-Intron Übergänge.
   B) Konservierte Nucleotid-Sequenzen des humanen Kv6.2 (SEQ ID NO: 1) und des Mus musculus Kv6.2 Gens (SEQ ID NO: 3), aus denen der offene Leserahmen abgeleitet wurde.
      Die Striche (-) in der Maus-Sequenz deuten auf zur humanen Sequenz identische Nukleotide hin.
   C) Offener Leserahmen der abgeleiteten Aminosäuresequenz (SEQ ID NO: 2) der humanen Kv6.2 α-Untereinheit.
      Die sechs hydrophoben, möglicherweise membranspannenden Segmente sind mit S1 bis S6 markiert. Die Markierungen mit PKC und CamK zeigen die putativen Phosphorylierungsstellen für Proteinkinase C und Ca²⁺-Calmodulin abhängige Kinase II.
   D) Homologie (in %) der humanen Kv6.2 Proteinsequenz (SEQ ID NO: 2) zu Proteinsequenzen von repräsentativen Mitgliedern der einzelnen Kv-Unterfamilien aus der Ratte.
Figur 2
   A) Northern-Analyse der Expression humaner Kv6.2 mRNA in verschiedenen Geweben.
      Die aufgetragene mRNA ist über der jeweiligen Spur vermerkt. Das humane genomische SacI/PstI-Fragment (SEQ ID NO: 5), das in Figur 1A als Probe A bezeichnet ist, wurde als Hybridisierungssonde verwendet. In Herz, Niere, Muskel, Leber und Pankreas wurde eine 5.5 kb große Kv6.2 mRNA detektiert. Die RNA-Menge in den einzelnen Bahnen wurde durch eine Hybridisierung mit einer markierten ß-Aktin cDNA Probe kontrolliert.
   B) Northern-Analyse der Expression der Kv6.2 mRNA in verschiedenen Geweben der Ratte.
      Die Herkunft der aufgetragenen mRNA ist über der jeweiligen Spur vermerkt. Ein genomisches DNA-Fragment (SEQ ID NO: 7), das die DNA-Sequenz von MKv6.2 zwischen Nucleotid 1281-1443 in Figur 1B beinhaltet, wurde hier als Hybridisierungssonde benutzt. Eine präferentiell im Herz exprimierte 2.6 kb lange mRNA wurde detektiert. Die RNA Menge in den einzelnen Bahnen wurde durch eine Hybridisierung mit einer markierten ß-Aktin cDNA Probe überprüft.
   C) Northern-Analyse der Expression der Kv6.2 mRNA innerhalb verschiedenen Regionen des Ratten-Herzens.
      Die aufgetragenen mRNAs aus verschiedenen Regionen des Ratten-Herzens sind über der jeweiligen Spur vermerkt. Die Kv6.2-Expression in Vorhofkammern ist stärker als in Septum und ventrikularen Kammern.
Figur 3
   *In situ* Hybridisierung und immunocytochemische Lokalisation des mKv6.2-Gens in adultem Maus-Gehirn.
   Eine 393 bp Antisense-RNA, die Nucleotide 1281-1443 der kodierenden mKv6.2 DNA (FIG. 1B(b), Seq. ID. Nr. 7) repräsentiert, wurde hier für die *in situ* Hybrisierung an adulten Maus-Gehirn-Schnitten verwendet.
   A) Präferentielle Expression der Kv6.2 mRNA in Körner-Zellen des Gyrus Dentatus und in Pyramiden-Zellen des hippocampalen CA3-Feldes.
   B) Kontrolle mit einer Sense-RNA Probe.
   C) Maus-Gehirn-Schnitt angefärbt mit dem Anti-Kv6.2 Antiköper, wobei eine starke Färbung im Moosfasersystem des Hippocampus gefunden wurde.
   D) Blockierung der in Figur 3C gezeigten Färbung durch Zugabe von Peptide (Kv6.2 Antigen).
Figur 4
   Lokalisation des Kaliumkanal-Gens, hKv6.2, in der Region des humanen Chromosoms 18q22-23.
Figur 5
   Vergleich der kinetischen Eigenschaften homomultimerer Kv2.1-Kanäle mit denen von Kanälen, die durch die Coexpression von Kv2.1 und Kv6.2 α-Untereinheiten gebildet werden.
   A) Auswärtsströme gemessen mit der 'patch-clamp'-Methode an mit hKv2.1 DNA (B. Albrecht et al., Receptor und Channels 1 (1995) 99, EMBL Access. Nr. L02840) oder mit hKv6.2-DNA (SEQ ID NO: 1) transient transfizierten CHO Zellen bzw. mit hKv2.1-DNA und hKv6.2-DNA cotransfizierten (hKv2.1 + hKv6.2) CHO-Zellen
   B) Auftragung normalisierter Leitfähigkeiten (G/Gmax, Ordinate) für humane Kv2.1-Ströme (gefüllte Kreise) und für coexprimierte humane Kv2.1-Ströme mit humanem Kv6.2 (offene Kreise) gegen das Membranpotential (Abzisse).
      Jeder Punkt stellt den Mittelwert ± Standardfehler von in sechs Experimenten für humanes Kv2.1 und elf Experimenten für das Kv2.1-Kv6.2 Heteromultimer durchgeführten Messungen dar. Durchgezogene Linien stellen die Ausgleichskurven gemäß der Boltzmann-Gleichung dar mit V_{0.5} = +10.8 ± 2.5 mV für Kv2.1 alleine und V_{0.5} = -10 ± 2.5 mV für Ströme für Kv2.1 koexprimiert mit Kv6.2 α-Untereinheiten (T-test für zwei Gruppen, p<0.005). Die Steigung zwischen beiden Kurven weist keinen Unterschied auf (S = 15.3 ± 1.5 für Kv2.1 Homomultimere; S = 14.5 ± 9 für Kv2.1/Kv6.2 Heteromultimere). Die Koexpression von hKv2.1 und hKv6.2 verschiebt den Spannungsverlauf der Aktivierung um 20 mV zu negativeren Testpotentialen gegenüber hKv2.1 allein.
   C) Effekt der hKv6.2 α-Untereinheit auf die Inaktivierungskinetik des Kv2.1-vermittelten Auswärtsstroms
      Die Wellenformen von Kaliumströmen in CHO-Zellen, die mit Kv2.1 alleine transfiziert worden waren, wurden mit jenen überlagert, die von CHO-Zellen, die mit Kv2.1- und Kv6.2-Untereinheiten kotransfiziert worden waren. Die Depolarisierungspulse wurden bis+40 mV angelegt. Das Haltepotential betrug -80 mV. Die Inaktivierungszeit-Konstante für Kv2.1-Ströme beträgt τ=7.17 ± 2.1 msec (n = 4), und für Kv2.1/Kv6.2 coexprimierte Ströme beträgt τ=4.98 ± 5 msec. (n = 4). Alle Transfektionen wurden in Gegenwart des Indikators GFP (grün fluoreszierendes Protein, M. Chalfie et al., Science 263 (1994) 802-805, S. Wang und T. Hazelrigg, Nature 369 (1994) 400-403) durchgeführt. Die Transfektionsabläufe und die Bedingungen für das Aufzeichnen von Strömen an ganzen Zellen waren dieselben wie die in Figur 6 beschriebenen.
Figur 6
   Die Koexpression von hKv2.1 und hKv6.2 verschiebt gegenüber hKv2.1 den Spannungsverlauf der Deaktivierung um 60mV zu negativeren Testpotentialen.

### Beispiele

### Beispiel 1: Isolierung von Klonen aus humanen und Maus genomischen DNA-Bibliotheken

1.10⁶ Plaques einer SVJ129 Maus genomischen DNA-Bibliothek (Clontech, Palo Alto, CA) wurden in 20 NZ-Platten (NZ: 16g/l NZ-Pulver, 5 g/l Hefeextrakt, 15 g/l Bacto-Agar; Chemikalien von Gibco BRL, Eggenstein, DE) mit einem Durchmesser 150 mm ausplattiert. Die genomische Phagen-DNA wurde dann auf 20 Membranfilter (Duralon-UV Membran, Stratagene) transferiert. Es folgte eine Hybridisierung der Filter in 50% Formamid, 0.8 M NaCl, 20 mM Pipes (Piperazin-N,N'-bis [2-ethansulfonsäure], Sigma), 1% SDS, 100µg/ml denaturierte Heringssperm-DNA, in H₂O und mit ³²P-markierter Maus Kv3.1 cDNA (Nucleotid 223-1356, Access. No. Y07521, Yokoyama et al., 1989) als Probe. Diese Hybridisierung wurde bei 60°C für 18 Stunden durchgeführt. Die Filter wurden anschließend mit 0.1xSET / 0.1% SDS in H₂O (20xSET: 3 M NaCl, 400 mM Tris/HCl, pH 7.4, 200 mM EDTA; Chemikalien von Sigma, für 0.1xSET wird 20xSet 1:200 verdünnt) gewaschen. Nach erfolgter Autoradiograhie bei -70°C wurden die auf den Röntgenfilmen (Kodak, Rochester, N.Y.) sichtbaren Signale den entsprechenden Plaques auf den Platten zugeordnet.

Die genomischen DNA-Fragmente wurden aus den positiven Phagen-Klonen isoliert und dann mit SacI, XbaI, EcoRI, BamHI und PstI sowohl einzeln als auch doppelt verdaut. Die verdauten DNA-Fragmente wurden in einem Agarose-Gel elektrophoretisch aufgetrennt und dann auf Nylonmembran transferiert. Drei DNA-Fragmente wurden durch eine weitere Hybridisierung, durchgeführt wie oben, identifiziert. Dies waren ein 1.0 kb BamHI/SacI Fragment, ein 1.0 kb XbaI/SacI Fragment und ein 0.9 kb SacI/PstI Fragment. Die Sequenzierungen der drei DNA-Fragmente zeigte, daß sie zusammen den gesamten kodierenden Bereich für Kv6.2 enthielten.

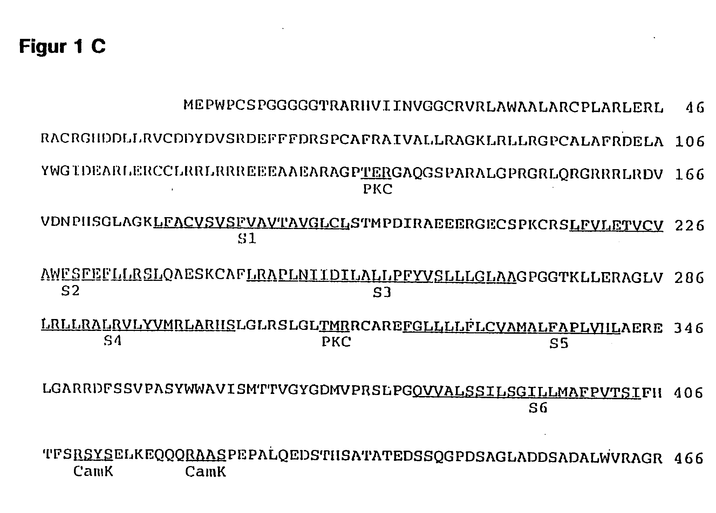

Die drei isolierten genomischen Maus DNA Fragmente wurden dann für die Isolierung des humanen Kv6.2-Gens als Hybridisierungssonden verwendet, wobei 1-10⁶ Phagen-Plaques aus einer humanen genomischen DNA-Bibliothek (Clontech, Palo Alto, CA) ausplattiert wurden. Die Hybridisierungs- und Waschbedingungen waren wie oben beschrieben. Nach Southern-Analyse und Sequenzierung wurden drei humane genomische Fragmente erhalten: ein 1.5 kb SacI/EcoRV Fragment, ein 0.8 kb PstI/SacI Fragment und ein 0.8 kb SacI/BamHI Fragment (Figur 1 A), die zusammen den gesamten kodierenden Bereich für Kv6.2 (Figur 1 C) enthielten.

### Beispiel 2: Kartierung der Restriktionsschnittsstellen innerhalb des isolierten genomischen DNA-Bereichs

Zur Erzeugung einer Restriktionskarte wurden die Insertionen der isolierten genomischen Phagen-DNAs mit Restriktionsenzymen nach Standardmethoden einfach oder doppelt verdaut und die Fragmente wurden anschließend in den Agarose-Gelen elektrophoretisch aufgetrennt (T. Maniatis et al., a.a.O.). Längenvergleiche der verdauten DNA-Fragmente ermöglichten die Erstellung der Restriktionskarten der isolierten genomischen Regionen (siehe Figur 1A). Die DNA-Fragmente, die den kodierenden Bereich des Kv6.2-Gens beinhalten, wurden mit dem Fachmann bekannten Methoden durch Southern-Analysen (T. Maniatis et al., a.a.O., E.M. Southern, J. Mol. Biol. 98(1975) 503-517) innerhalb der isolierten genomischen Regionen lokalisiert. Die Sequenzierungen dieser DNA-Fragmente ermöglichten die Identifizierung der Transkriptionsrichtung des Kv6.2-Gens.

### Beispiel 3: DNA-Sequenzierung

Die DNA-Fragmente, die dem kodierenden Bereich des Kv6.2.Gens entsprachen, wurden in die EcoRI-Schnittstellen des *Bluescript* Vektors (Stratagene, La Jolla, CA) kloniert. Die Kv6.2 DNA wurde dann nach der Methode von Sanger et al., (P.N.A.S. USA 74 (1977) 5463-5467) mit T7-DNA Polymerase (Sequenase, US Biochemicals, Cleveland, Ohio) sequenziert. Plasmid-spezifische Oligonukleotide M13, Reverse, T3 und T7 (Stratagene, La Jolla, CA, SEQ ID Nos: 23-26) wurden für die Sequenzierungen als Primer verwendet.

### Beispiel 4: Northern-Analyse

Der *Multiple Tissue Northern (MTN) blot* (Clontech, Palo Alto, CA) enthielt jeweils 2 mg poly-A⁺ mRNA aus Herz, Gehirn, Plazenta, Lunge, Leber, Skelettmuskel, Niere und Pankreas. Das 0.6 kb SacI/PstI-Fragment (Probe A in Figur 1 A) wurde ³²P-markiert (T. Maniatis et al., a.a.O.) und dann als Hybridisierungssonde verwendet. Die Sonde wurde in Hybridisierungslösung (50% Formamid, 5XSET, 10x Denhardt's (100x Denhardt's: 20 g/l Ficoll 400 (Sigma), 20 g/l BSA (Sigma), 20 g/l Polyvinylpyrolidon (Merck), 1:10 verdünnt), 1% SDS (Biorad), 100mg/ml Heringssperm DNA (Sigma) in H₂O) bei 42°C 24 Stunden an die mRNA hybridisiert. Der Blot wurde anschließend nacheinander in Waschlösung 1 (2XSSC (20xSSC: 173 g/l NaCl, 88,2 g/l NaCitrat, pH 7,0, alle Chemikalien von Sigma, für 2XSSC 1:10 verdünnt)); 0.1% SDS; in H₂O) bei RT und in Waschlösung 2 (0.1XSSC (20XSSC 1:200 verdünnt; 0.1% SDS; in H₂O) bei 50°C gewaschen. Nach dem Waschen erfolgte eine Autoradiograhie auf den Röntgenfilmen (Kodak, Rochester, N.Y.) bei -70°C.

Der zweite *Multiple Tissue Northern (MTN) blot* (Clontech, Palo Alto, CA) enthielt jeweils 2 mg poly-A⁺ mRNA aus Ratten-Herz, - Gehirn, -Milz, -Lunge, -Leber, -Skelettmuskel, -Niere und - Testis. Der *Northern blot* mit RNA aus Ratten-Herz enthielt jeweils 5 mg poly-A⁺ mRNA aus linkem Vorhof, rechtem Vorhof, Septum, linker ventrikularer Kammer und rechter ventrikularer Kammer. Das 1.2 kb SacI/SacI genomische Maus DNA-Fragment wurde ³²P-markiert und dann als Hybridisierungssonde für die beiden *Blots* verwendet. Die Hybridisierungs- und Waschbedingungen sowie die Autoradiographiebedingungen entsprachen denen des ersten MTN-Blots.

### Beispiel 5: PCR

Die Verknüpfung der zwei kodierenden Bereiche des humanen Kv6.2-Gens wurde durch eine Kombination von PCR-Technik und Verdauung mit der Typ IIS Restriktionsendonuklease Eam 1104I durchgeführt (K.A. Padgett et al., Gene 168 (1996) 31-35). Der erste 624 bp kodierende Bereich für N-Terminus und S1-Segment (Nukleotide 1-624 von Seq. ID. Nr. 1) wurde durch PCR mit zwei Oligonukleotiden *Seam1* (SEQ ID NO: 17) und *Seam6* (SEQ ID NO:19) amplifiziert, wobei das 1.2 kb große genomische humane SacI/EcoRV DNA-Fragment als Matrize verwendet wurde. Eine Kozak-Sequenz (5'-CCACC-3', SEQ ID NO: 27) wurde vor das Start-Codon in das *Seam1* Oligonukleotid eingebaut. Der zweiter 777 bp kodierender Bereich für S2-S6 Segmente und C-Terminus (Nukleotide 625-1401 von Seq. ID. Nr. 1) wurde durch eine zweite PCR mit Oligonukleotiden *Seam5* (SEQ ID NO: 20) und *Seam 4* (SEQ ID NO: 22) amplifiziert, wobei das 1.6 kb PstI/BamHI genomische humane DNA-Fragment (Figur 1 A) als Matrize verwendet wurde. Für die beiden Amplifikationen wurde insgesamt 14 Reaktionszyklen mit *KlenTaq* Polymerase (Clontech, Palo Alto, CA) durchgeführt, wobei die methylierten Deoxycytosintriphosphate (dCTPs, Stratagene) in das DNA-Fragment durch Zugabe von 5'-Methyl-dCTP in den letzten fünf Reaktion-Zyklen eingebaut wurden. Die anschließende Verdauung mit Eam 1104I (Stratagene, La Jolla, CA) erzeugte komplementäre überhängende Enden am 3'-Ende des ersten PCR-Fragments (CCC) und am 5'-Ende des zweiten PCR-Fragments (GGG). Das eingebaute methylierte dCTP verhinderte die Verdauung der internen Eam 1104I Schnittstellen in den PCR-Fragmenten. Durch Ligation mit T4-DNA-Ligase (MBI Fermentas, Buffalo, NY) wurden die beiden PCR-Fragmente miteinander verknüpft. Es resultierte ein DNA-Fragment mit dem gesamten kodierenden Bereich des humanen Kv6.2-Gens.

### Beispiel 6: Humane chromosomale Lokalisation

Ein 14 kb langer humaner genomischer λDNA-Klon, der den kodierenden Bereich für S2-S6 und C-Terminus der Kv6.2 α-Untereinheit enthielt, wurde mit Biotin-16-dUTP (Boehringer, Mannheim) markiert und dann für eine FISH-Analyse als Sonde verwendet. Die FISH-Analyse erfolgte nach der von P. Lichter et al., PNAS USA 85 (1988) 9664-9668 und C. Fonatsch et al., Int. J. Cancer 26 (1980) 749-754 beschriebenen Methode. Die Signale wurden mit Fluoreszenz-Isothiozyanat gekoppeltem Avidin-DCS^{®} (Vector Laboratories) detektiert und die Lokalisierung der Signale in Metaphase-Chromosomen wurde mit Hilfe eines *konfocalen Laser Scanning* Mikroskops (C. Zeiss, LSM 410, Germany) durchgeführt.

### Beispiel 7: In situ Hybridisierung und Immunozytochemie

Das 393 bp lange DNA-Fragment (Nukleotide 1-393 von SEQ ID NO: 7) aus dem 0.9 kb Maus SacI/pstI-DNA-Fragment wurde in den Blueskript Vektor umkloniert. Zwei linealisierte DNA-Klone mit dieser Insertion in zwei verschiedenen Orientierungen wurden für die Synthesen von Anti-Sense-RNA und Sense-RNA mit Hilfe des *mMessagemMachine* Kits (Ambion, Austin, TX) verwendet, wobei die RNAs mit Hilfe von T₃- bzw. T₇-RNA-Polymerase synthetisiert und dabei mit ³³P-UTP markiert wurden (Melton et al., Nucleic Acids Res. 12 (1984) 7035-7056).

In einem Kryostaten wurden 10-16 µm dicke Gefrierschnitte angefertigt und bei Raumtemperatur getrocknet. Anschließend wurden die Schnitte für 5 Min. in eiskalter, PBS gepufferter, 4 %ige Formalin-Lösung fixiert. Die Hybridisierungen mit den ³³P-markierten Antisense- und Sense-RNAs erfolgten in Hybrisierungslösung (50 % Formamid (Fluka), 10 % Dextransulfat (Sigma), 0.3 M NaCl (Sigma), 20 mM Tris/HCl (Sigma, pH=7.4), 5mM EDTA (Sigma), 20 mM DTT (Dithiotreitol, Sigma), 1x Denhardt's Reagenz (100x Denhardt's 1:100 verdünnt), 100µg/ml denaturierte Lachssperma-DNA, 200 µg/ml Hefe-tRNA) unter einem Deckgläschen über Nacht bei 42°C. Die Schnitte wurden dann in lxSSC/4 mM DTT bei 55-65°C gewaschen. Die Objektträger wurden dann über 1xSSC/ 4 mM DTT, 0.1xSSC, 75 % Ethanol entwässert und luftgetrocknet. Die Exposition erfolgte für 3-7 Tage bei Raumtemperatur mit MR-Film (Kodak, Rochester, NY. Die Schnitte wurden dann in auf 42°C vorgewärmte Photoemulsion (Kodak, Rochester, NY) getaucht, über Nacht bei Raumtemperatur getrocknet und für 1 Woche bei 4°C exponiert. Die Entwicklung erfolgte mit D19-Entwickler und Unifix (Kodak, Rochester, NY).

Für die Erzeugung der Antigen-Peptide wurde das 393 bp DNA-Fragment (Nukleotide 1-393 von SEQ ID NO: 7) aus dem 0.9 kb genomischen SacI/PstI Maus-DNA-Fragment isoliert und dann in den kodierenden Leserahmen des *Glutathion-S-Transferase* (GST) Gens im *pGEX-2T* Vektor (D.B. Smith und K.S. Johnson, Gene 67 (1988) 31-40) umkloniert. Dieses DNA-Fragment enthielt den kodierenden Bereich für den C-Terminus der mKv6.2 α-Untereinheit (C-mKv6.2, SEQ ID NO: 8). Das Fusionsprotein von GST und C-mKv6.2 wurde durch Zugabe von 1 mM IPTG (Isopropylthiogalactosid, Gibco-BRL) in dem mit dieser Plasmid-DNA transformierten E.coli-Bakterienstamm *XL-1 Blue* (Stratagene) induziert und dann durch *Glutathion-Agarose* (Sigma, St. Louis, MO) aufgereinigt. Zwei ca. 4-5 Monate alte weibliche Kaninchen wurden mit diesem Fusionsprotein nach der Standard-Methode (E. Harlow und D. Lane, a.a.O.) immunisiert. Für die Affinitätsreinigung des Anti-Kv6.2 Antikörpers wurde C-mKv6.2 mit einem His-Tag im Bakterienstamm BL21 induziert, der den pET-16b Vektor (Novagen, Madison, WI, F.W. Studier et al., Methods in Enzymology 185 (1990) 60-89) mit dem 393 bp DNA-Fragment (Nukleotide 1-393 von Seq. ID. Nr. 7) enthielt. Die Aufreinigung des C-mKv6.2-Proteins mit His-Tag wurde durch eine Nickel-Säule (Novagen, Madison, WI) durchgeführt. Ca. 100 µg aufgereinigtes C-mKv6.2-Protein wurde auf eine 1 cm² große Nitrocellulosemembran (Schleicher & Schuell, Keen, NH) gebunden und dann zwei Stunden lang mit 1xPBS Lösung mit 5 % Milchpulver bei Raumtemperatur blockiert. Die erhaltene Membran wurde in der PBS-Lösung mit 1:5 verdünntem Kaninchen-Antiserum über Nacht bei 4°C inkubiert und dann einmal in der Waschlösung 1 (1xPBS (20XPBS: 3 M NaCl, 161 mM Na₂HPO₄, 39 mM KH₂PO₄, 1:20 verdünnt), 1 % BSA (Sigma), 0.5 % Triton X-100 (Sigma)) und zweimal in der Waschlösung 2 (1xPBS, 1 % BSA) gewaschen. Die Elution erfolgte in einer Elutionslösung (0.2 M Glycin, 0.15 M NaCl, 0.1 % BSA, pH=2.5).

Adulte Mäuse wurden betäubt und mit einer Lösung aus 4 % Formaldehyd, 0.05 % Glutaraldehyd. und 0.2 % Pikrinsäure in 0.1 M Phosphatpuffer (pH=7.4) perfundiert. Nach der Fixierung wurde mit 0.15 M Saccharose in 0.13 M Phosphatpuffer (pH=7.4) gespült, das Gehirn wurde herausoperiert und auf -50°C eingefroren. 25 µm dicke Gefrierschnitte wurden dann bei -21°C angefertigt. Die Schnitte wurden 30 min bei Raumtemperatur mit 1 % NaBH₄ in PBS reduziert und dann mit PBS gewaschen. Zur Zerstörung der endogenen Peroxidaseaktivität wurden die Schnitte für 30 Minuten bei RT mit 0.05 % Phenylhydrazin und 10 % normales Ziege-Serum (Gibco-BRL) mit 0.3 % Triton x-100 behandelt. Die Schnitte wurden mit dem 1:50 verdünnten gereinigten Anti-C-mKv6.2 Antikörper bei 4°C über Nacht inkubiert. Für das Peptid-Blockierungsexperiment wurde dieser Antikörper in einer 10 µg/ml Antigen-Peptidlösung für 2 Stunden bei Raumtemperatur präinkubiert. Nach dem Waschen mit PBS wurden die Schnitte weitere 3 Stunden bei Raumtemperatur mit der Zweitantikörperlösung (biotinylierter Ziege-Kaninchen Antikörper, 1:2000 in PBS, Camon) inkubiert und anschließend mit dem Elite-ABC-Komplex (Vectastain Elite-ABC Kit, Vector) für 1 Stunde bei Raumtemperatur inkubiert. Die Farbreaktion mit 3,3'-Diaminobenzidin wurde in 0.015 % H₂O₂ für 3 min durchgeführt (S.M. Hsu et al., J. Histochem. Cytochem. 29 (1981) 577-580).

### Beispiel 8: Funktionelle Expression und elektrophysiologische Technik

Das DNA-Fragment (SEQ ID NO: 1), das den gesamten kodierenden Bereich für die hKv6.2 α-Untereinheit enthielt, wurde in den dem Fachmann gut bekannten pCDNA3 Vektor (Invitrogene, Carlsbad, CA) zu Expressionsstudien kloniert. hKv2.1 cDNA auch in einen anderen pCDNA3 Vektor (Invitrogene, Carlbad, CA) inseriert. Insgesamt 1µg Plasmid-DNA (entweder Kv2.1, Kv6.2 allein oder Kv2.1 mit Kv6.2 zusammen) und 1µg DNA für GFP wurde zur Transfektion von CHO-Zellen mit DMRIE-C-Reagens (eine 1:1 Mischung aus Kation-Lipid DMRIE und Cholesterol, Gibco-BRL, Life Technologies) eingesetzt. 500ml Opti-MEM 1-Medium (Gibco-BRL, Life Technologies) mit 2 µg Plasmid-DNA und 500 ml Opti-MEM 1-Medium mit 6.4 µl DMRIE-C-Reagens wurden zusammengemischt, und anschließend bei RT für 45 Minuten inkubiert, wobei ein Liposom-DNA-Komplex gebildet wurde. 2·10⁵ CHO-Zellen in einer 35-mm Schale wurden zuerst mit Opti-MEM 1-Medium gewaschen, danach wurde die Lösung mit diesem Lipid-DNA-Komplex auf die Zellschicht ausplattiert. Nach einer Inkubation für 5-6 Stunden bei 37°C in einem Inkubator unter 5% CO₂ wurde die Lösung durch normales Medium ersetzt, und die Zellen wurden für weitere 12-24 Stunden bei 37°C inkubiert. Anschließend wurden 5·10⁴ Zellen in eine neue Schale (35 mm) für die elektrophysiologischen Messungen umgesetzt.

Auswärtsströme wurden 24-48 Stunden nach der Transfektion mit Hilfe der *whole-cell* Konfiguration der *patch-clamp* Technik (O.P. Hamill et al., *Pflügers Arch.* (1981) 85-100) gemessen. Die Mikropipetten wurden mit einem DMZ-Universal Puller (Zeitz Instruments, Augsburg, Germany) gezogen und hatten einen Widerstand zwischen 2-3 MΩ nach der Füllung mit der intrazelluraren Lösung (105 mM Kaliumaspartat, 20 mM KCl 10 mM BAPTA, 10 mM HEPES, 1.5 mM MgCl₂, 2 mM CaCl₂, 5mM Glukose, 2 mM ATP-Na₂, pH=7.2, alle Chemikalien von Sigma). Für die elektrophysiologischen Messungen wurden die CHO-Zellen zuerst in einer extrazellularen Lösung (140mM NaCl, 5.3 KCl, 1 mM MgCl₂, 2 mM CaCl₂, 10 mM HEPES, 5 mM Glukose, pH=7.3) auf einem Haltepotential -80 mV gehalten und anschließend depolarisiert auf Testpotentiale von - 70 bis 80 mV in Abstände von 10 mV von je 300 ms Dauer.

## Patentansprüche

1. Kaliumkanalprotein, **dadurch gekennzeichnet, daß** es ein in Leber, Skelettmuskel, Niere, Pankreas, Gehirn, Lunge oder Placenta exprimiertes Homologes, ein Derivat oder ein Fragment eines Kaliumkanalproteins mit einer Aminosäuresequenz nach SEQ ID NO: 2 oder 4 ist, das die gleiche elektrophysiologische, pharmakologische und/oder biologische Wirkung und/oder Immunogenität aufweist, mit der Maßgabe, dass das Homologe nicht die Sequenz 
MALLTGNADR AFSSYSFNKL ENLCEVQTKK GFFYRKAKLL HPDEDLCYLA RLDDRTRFVI INVGGIKYKV PWTTLENCPL
TRLGKLKSCN NYDEIMNICD DYDVSCNEFF FDRNPSAFRT IMTFLTAGKL RLLREMCALS FQEELVYWGI EEDHLEWCCK
KRLQQKEEEA AEARMYEGEM MFSETTQCAF QDNNWLSLCM RNLRDMVENP HSGIPGKIFA CISISFVAIT AVSLCISTMP
DVREEEDRGE CSQKCYDIFV LETVCVAWFS FEFLLRSIQA ENKCAFLKTP LNIIDILAIL PFYISLIVDM ASTKNSSKPG
GGAGNKYLER VGLVLRFLRA LRILYVMRLA RHSLGLQTLG LTVRRCTREF GLLLLFLCVA MALFSPLVYL AESELGAKQE
FTSIPTSYWW AVISMTTVGY GDMVPRSIPG QVVALSSILS GILIMAFPVT SIFHTFSRSY SELKEQQQRA ASRQKHQLE
STKLAGGGSS QWITAASPPD AAREDGRPEL DQEAKRSC
 aufweist.

2. Kaliumkanalprotein nach Anspruch 1, **dadurch gekennzeichnet, daß** es eine Aminosäureidentität von mindestens 60 % zum humanen Kaliumkanalprotein (SEQ ID NO: 2) aufweist und einer vom Menschen verschiedenen Spezies entstammt.

3. Kaliumkanal, **dadurch gekennzeichnet, daß** er mindestens das Kaliumkanalprotein nach einem der Ansprüche 1 oder 2 enthält.

4. Kaliumkanal nach Anspruch 3, **dadurch gekennzeichnet, daß** er zusätzlich das Kaliumkanalprotein Kv2.1 enthält.

5. Kaliumkanal nach Anspruch 3 oder 4, **dadurch gekennzeichnet, daß** er ein spannungsabhängiger Kaliumkanal ist.

6. Nukleinsäuremolekül, **dadurch gekennzeichnet, daß** es für ein Kaliumkanalprotein nach den Ansprüchen 1 bis 2 kodiert.

7. Nukleinsäuremolekül, **dadurch gekennzeichnet, daß** es für einen Kaliumkanal nach den Ansprüchen 3 bis 5 kodiert.

8. Nukleinsäuremolekül nach Anspruch 6 oder 7, **dadurch gekennzeichnet, daß** es eine Sequenz enthält, die aus der Gruppe bestehend aus
allelischen Varianten und Fragmenten der Sequenzen SEQ ID NO: 1 oder 3, soweit sie für ein Protein mit gleicher elektropysiologischer, pharmakologischer und/oder biologischer Wirksamkeit und/oder Immunogenität kodieren.

9. Vektor, **dadurch gekennzeichnet, daß** er eines oder mehrere der Nukleinsäuremoleküle nach den Ansprüchen 6 bis 8 enthält.

10. Vektor nach Anspruch 9, **dadurch gekennzeichnet, daß** er ein Expressionsvektor ist.

11. Wirtszelle, **dadurch gekennzeichnet, daß** sie mit einem Vektor nach den Ansprüchen 9 oder 10 transformiert ist.

12. Wirtszelle nach Anspruch 11, **dadurch gekennzeichnet, daß** sie mit einem weiteren Vektor, der ein Nukleinsäuremolekül enthält, das für ein weiteres Kaliumkanalprotein kodiert, transformiert ist.

13. Wirtszelle nach Anspruch 12, **dadurch gekennzeichnet, daß** der weitere Vektor ein Nukleinsäuremolekül enthält, das für das Kaliumkanalprotein Kv2.1 kodiert.

14. Wirtszelle nach den Ansprüchen 11 bis 13, **dadurch gekennzeichnet, daß** sie eine CHO-Zelle oder ein Xenopus-Oozyt ist.

15. Wirtszelle nach den Ansprüchen 11 bis 14, **dadurch gekennzeichnet, daß** sie den durch die Vektoren kodierten Kaliumkanal exprimiert.

16. Wirtszelle nach Anspruch 15, **dadurch gekennzeichnet, daß** sie den durch die Vektoren kodierten Kaliumkanal auf ihrer Oberfläche exprimiert.

17. Verfahren zur Expression eines Kaliumkanals, **dadurch gekennzeichnet, daß** man eine eukaryontische Wirtszelle nach den Ansprüchen 11 bis 16 unter Bedingungen kultiviert, die zur Expression des Kaliumkanals geeignet sind.

18. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, **dadurch gekennzeichnet, daß** man
a) an Wirtszellen nach den Ansprüchen 11 bis 16 den Kaliumauswärtstrom mißt,
b) die Wirtszellen mit einer Substanz in Kontakt bringt und
c) an Wirtszellen erneut den Kaliumauswärtsstrom mißt,
wobei der Unterschied zwischen dem Kaliumauswärtsstrom vor und nach Zugabe der Substanz die Aktivität der Substanz bestimmt.

19. Verfahren nach Anspruch 18, **dadurch gekennzeichnet, daß** man den Kaliumauswärtsstrom mit Hilfe der 'patch-clamp'-Methode bestimmt.

20. Verfahren nach den Ansprüchen 18 oder 19, **dadurch gekennzeichnet, daß** eine Substanz eine öffnende Substanz ist, wenn nach Zugabe der Substanz bei einem Membranpotential, bei dem ohne Zugabe der Substanz keine Kaliumauswärtsströme fließen, Kaliumauswärtsstöme fließen.

21. Verfahren nach den Ansprüchen 18 oder 19, **dadurch gekennzeichnet, daß** eine Substanz eine aktivierende Substanz ist, wenn nach Zugabe der Substanz ein bereits vorhandener Kaliumauswärtsstrom verstärkt wird.

22. Verfahren nach den Ansprüchen 18 oder 19, **dadurch gekennzeichnet, daß** eine Substanz eine schließende Substanz ist, wenn nach Zugabe der Substanz bei einem Membranpotential, bei dem ohne Zugabe der Substanz Kaliumauswärtsströme fließen, keine Kaliumauswärtsströme fließen.

23. Verfahren nach den Ansprüchen 18 oder 19, **dadurch gekennzeichnet, daß** eine Substanz eine inaktivierende Substanz ist, wenn nach Zugabe der Substanz ein bereits vorhandener Kaliumauswärtsstrom vermindert wird, ohne daß der Kaliumasuwärtsstrom vollständig zum Erliegen kommt.

24. Verfahren nach den Ansprüchen 18 oder 19, **dadurch gekennzeichnet, daß** eine Substanz eine verändernde Substanz ist, wenn durch Zugabe der Substanz biophysikalische Eigenschaften des Kaliumkanals wie Spannungsabhängigkeit, Leitfähigkeit, Aktivierungszeitkonstanten, Inaktivierungszeitkonstanten, Schaltverhalten, Offenzeiten oder Geschlossenzeiten verändert werden.

25. Verfahren nach den Ansprüchen 18 oder 19, **dadurch gekennzeichnet, daß** eine Substanz eine verändernde Substanze ist, wenn durch Zugabe der Substanz die Zelloberflächenexpression des Kaliumkanals verändert wird.

26. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, **dadurch gekennzeichnet, daß** man
a) an Wirtszellen nach den Ansprüchen 11 bis 16 das Membranpotential mißt,
b) die Wirtszellen mit einer Substanz in Kontakt bringt und
c) an Wirtszellen erneut das Membranpotential mißt,
wobei der Unterschied zwischen dem Membranpotential vor und nach Zugabe der Substanz die Aktivität der Substanz bestimmt.

27. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, bei dem man
a) an Wirtszellen nach den Ansprüchen 11 bis 16 das Membranpotential und den Kaliumauswärtsstrom mißt,
b) die Wirtszellen mit einer Substanz in Kontakt bringt und
c) das Membranpotential und den Kaliumauswärtsstrom erneut mißt,
wobei die Unterschiede zwischen dem Membranpotential und dem Kaliumauswärtsstrom vor und nach Zugabe der Substanz die Aktivität der Substanz bestimmt.

28. Antikörper, der an das isolierte Kaliumkanalprotein nach den Ansprüchen 1 oder 2 bindet.

29. Antikörper, der an das Kaliumkanalprotein nach den Ansprüchen 1 oder 2 bindet, wobei das Kaliumkanalprotein Bestandteil eines Kaliumkanales nach den Ansprüchen 3 bis 5 ist.

30. Antikörper nach Anspruch 28 oder 29, **dadurch gekennzeichnet, daß** er ein polyklonaler oder monoklonaler Antikörper ist.


SEQUENZPROTOKOLL

<110> Evotec AG

<120> Neuer spannungsabhängiger Kaliumkanal und seine
      Verwendung zur Entwicklung von Therapeutika

<130> P76936

<140> EP 99941625.8
<141> 1999-08-06

<150> PCT/EP99/05983
<151> 1999-08-06

<150> DE 198 41 413.7
<151> 1998-08-06

<160> 28

<170> PatentIn Vers. 2.0

<210> 1
<211> 1401
<212> DNA
<213> Homo sapiens

<220>
<221> CDS
<222> (1)..(1401)
<223> Reifes humanes Kv6.2 Protein

<400> 1
atg gag cca tgg ccc tgc tcc ccg ggc ggc ggc ggc ggg acc cgc gcc     48
Met Glu Pro Trp Pro Cys Ser Pro Gly Gly Gly Gly Gly Thr Arg Ala
  1               5                  10                  15

cgg cac gtc atc atc aac gtg ggc ggc tgc cgc gtg cgc ctg gca tgg     96
Arg His Val Ile Ile Asn Val Gly Gly Cys Arg Val Arg Leu Ala Trp
             20                  25                  30

gcc gcg ctg gcg cga tgc ccc ctc gcg cgc ctg gag cgc ctg cgc gcc     144
Ala Ala Leu Ala Arg Cys Pro Leu Ala Arg Leu Glu Arg Leu Arg Ala
         35                  40                  45

tgc cgc ggc cac gac gac ctg ctg cgc gtg tgt gac gac tac gac gtg     192
Cys Arg Gly His Asp Asp Leu Leu Arg Val Cys Asp Asp Tyr Asp Val
     50                  55                  60

agc cgc gac gag ttc ttc ttc gac cgc agc ccg tgc gcc ttc cgc gcc     240
Ser Arg Asp Glu Phe Phe Phe Asp Arg Ser Pro Cys Ala Phe Arg Ala
 65                  70                  75                  80

atc gtg gcg ctt ttg cgc gca ggg aag ctg cga ctg ctg cgg ggc ccg     288
Ile Val Ala Leu Leu Arg Ala Gly Lys Leu Arg Leu Leu Arg Gly Pro
                 85                  90                  95

tgc gcg ctg gcc ttc cgc gac gag ctg gcc tac tgg ggc atc gac gag     336
Cys Ala Leu Ala Phe Arg Asp Glu Leu Ala Tyr Trp Gly Ile Asp Glu
            100                 105                 110

gcg cgc ctg gag cgc tgc tgc ctg cgc cgc ctg cgc cgc cgc gag gag     384
Ala Arg Leu Glu Arg Cys Cys Leu Arg Arg Leu Arg Arg Arg Glu Glu
        115                 120                 125

gag gcg gcc gag gcc cgc gcg ggg ccg acg gag cgc ggg gcg cag ggg     432
Glu Ala Ala Glu Ala Arg Ala Gly Pro Thr Glu Arg Gly Ala Gln Gly

130                 135                 140

agc ccg gcg cgc gcc ctg gga cct cgg ggg cgg ctg cag cgc ggc cgg     480
Ser Pro Ala Arg Ala Leu Gly Pro Arg Gly Arg Leu Gln Arg Gly Arg
145                 150                 155                 160

cgg cgc ctg cgc gac gtg gtg gac aac ccg cac tcg ggg ctg gcg ggc     528
Arg Arg Leu Arg Asp Val Val Asp Asn Pro His Ser Gly Leu Ala Gly
                165                 170                 175

aag ctc ttc gcc tgc gtg tcc gtg tcc ttc gtg gcc gtc acg gcc gtg     576
Lys Leu Phe Ala Cys Val Ser Val Ser Phe Val Ala Val Thr Ala Val
            180                 185                 190

ggc ctc tgc ctg agc acc atg ccg gac atc cgc gcc gag gag gag cgg     624
Gly Leu Cys Leu Ser Thr Met Pro Asp Ile Arg Ala Glu Glu Glu Arg
        195                 200                 205

ggc gag tgc tcc ccc aag tgc cgc agc ctg ttc gtg ctg gag acc gtg     672
Gly Glu Cys Ser Pro Lys Cys Arg Ser Leu Phe Val Leu Glu Thr Val
    210                 215                 220

tgc gtg gcc tgg ttc tcc ttc gag ttc ctg ctg cgc tcc ctg cag gcc     720
Cys Val Ala Trp Phe Ser Phe Glu Phe Leu Leu Arg Ser Leu Gln Ala
225                 230                 235                 240

gag agc aag tgc gcc ttc ctg cgc gcg cca ctc aac atc att gac atc     768
Glu Ser Lys Cys Ala Phe Leu Arg Ala Pro Leu Asn Ile Ile Asp Ile
                245                 250                 255

ctg gcg ctc ctg ccg ttc tac gtg tcg ctg ctg ctg ggg ctg gcg gca     816
Leu Ala Leu Leu Pro Phe Tyr Val Ser Leu Leu Leu Gly Leu Ala Ala
            260                 265                 270

ggc ccg ggc ggg acc aag ctc ctg gag cgc gcg ggg ctg gtg ctg cgg     864
Gly Pro Gly Gly Thr Lys Leu Leu Glu Arg Ala Gly Leu Val Leu Arg
        275                 280                 285

ctg ctg cgt gcg ctg cgc gtg ctc tac gtg atg cgc ctg gcg cgc cac     912
Leu Leu Arg Ala Leu Arg Val Leu Tyr Val Met Arg Leu Ala Arg His
    290                 295                 300

tcg ctg ggg ctg cgt tcg ctg ggc ctg acc atg cgc cgc tgc gcg cgc     960
Ser Leu Gly Leu Arg Ser Leu Gly Leu Thr Met Arg Arg Cys Ala Arg
305                 310                 315                 320

gag ttc ggg ctg ctg ctg ctg ttc ctc tgc gtg gcc atg gcg ctc ttc     1008
Glu Phe Gly Leu Leu Leu Leu Phe Leu Cys Val Ala Met Ala Leu Phe
                325                 330                 335

gcg cca ctg gtg cac ctg gcc gag cgc gag ctg ggc gcg cgc cgc gac     1056
Ala Pro Leu Val His Leu Ala Glu Arg Glu Leu Gly Ala Arg Arg Asp
            340                 345                 350

ttc tcc agc gtg ccc gcc agc tat tgg tgg gcc gtc atc tcc atg acc     1104
Phe Ser Ser Val Pro Ala Ser Tyr Trp Trp Ala Val Ile Ser Met Thr
        355                 360                 365

acc gtg ggc tac ggc gac atg gtc ccg cgc agc ctg ccc ggg cag gtg     1152
Thr Val Gly Tyr Gly Asp Met Val Pro Arg Ser Leu Pro Gly Gln Val
    370                 375                 380

gtg gcg ctc agc agc atc ctc agc ggc atc ctg ctc atg gcc ttc ccg     1200
Val Ala Leu Ser Ser Ile Leu Ser Gly Ile Leu Leu Met Ala Phe Pro
385                 390                 395                 400

gtc acc tcc atc ttc cac acc ttt tcg cgc tcc tac tcc gag ctc aag     1248

Val Thr Ser Ile Phe His Thr Phe Ser Arg Ser Tyr Ser Glu Leu Lys
                405                 410                 415

gag cag cag cag cgc gcg gcc agc ccc gag ccg gcc ctg cag gag gac     1296
Glu Gln Gln Gln Arg Ala Ala Ser Pro Glu Pro Ala Leu Gln Glu Asp
            420                 425                 430

agc acg cac tcg gcc aca gcc acc gag gac agc tcg cag ggc ccc gac     1344
Ser Thr His Ser Ala Thr Ala Thr Glu Asp Ser Ser Gln Gly Pro Asp
        435                 440                 445

agc gcg ggc ctg gcc gac gac tcc gcg gat gcg ctg tgg gtg cgg gca     1392
Ser Ala Gly Leu Ala Asp Asp Ser Ala Asp Ala Leu Trp Val Arg Ala
    450                 455                 460

ggg cgc tga                                                         1401
Gly Arg
465


<210> 2
<211> 466
<212> PRT
<213> Homo sapiens

<400> 2
Met Glu Pro Trp Pro Cys Ser Pro Gly Gly Gly Gly Gly Thr Arg Ala
  1               5                  10                  15

Arg His Val Ile Ile Asn Val Gly Gly Cys Arg Val Arg Leu Ala Trp
             20                  25                  30

Ala Ala Leu Ala Arg Cys Pro Leu Ala Arg Leu Glu Arg Leu Arg Ala
         35                  40                  45

Cys Arg Gly His Asp Asp Leu Leu Arg Val Cys Asp Asp Tyr Asp Val
     50                  55                  60

Ser Arg Asp Glu Phe Phe Phe Asp Arg Ser Pro Cys Ala Phe Arg Ala
 65                  70                  75                  80

Ile Val Ala Leu Leu Arg Ala Gly Lys Leu Arg Leu Leu Arg Gly Pro
                 85                  90                  95

Cys Ala Leu Ala Phe Arg Asp Glu Leu Ala Tyr Trp Gly Ile Asp Glu
            100                 105                 110

Ala Arg Leu Glu Arg Cys Cys Leu Arg Arg Leu Arg Arg Arg Glu Glu
        115                 120                 125

Glu Ala Ala Glu Ala Arg Ala Gly Pro Thr Glu Arg Gly Ala Gln Gly
    130                 135                 140

Ser Pro Ala Arg Ala Leu Gly Pro Arg Gly Arg Leu Gln Arg Gly Arg
145                 150                 155                 160

Arg Arg Leu Arg Asp Val Val Asp Asn Pro His Ser Gly Leu Ala Gly
                165                 170                 175

Lys Leu Phe Ala Cys Val Ser Val Ser Phe Val Ala Val Thr Ala Val
            180                 185                 190

Gly Leu Cys Leu Ser Thr Met Pro Asp Ile Arg Ala Glu Glu Glu Arg
        195                 200                 205

Gly Glu Cys Ser Pro Lys Cys Arg Ser Leu Phe Val Leu Glu Thr Val
    210                 215                 220

Cys Val Ala Trp Phe Ser Phe Glu Phe Leu Leu Arg Ser Leu Gln Ala
225                 230                 235                 240

Glu Ser Lys Cys Ala Phe Leu Arg Ala Pro Leu Asn Ile Ile Asp Ile
                245                 250                 255

Leu Ala Leu Leu Pro Phe Tyr Val Ser Leu Leu Leu Gly Leu Ala Ala
            260                 265                 270

Gly Pro Gly Gly Thr Lys Leu Leu Glu Arg Ala Gly Leu Val Leu Arg
        275                 280                 285

Leu Leu Arg Ala Leu Arg Val Leu Tyr Val Met Arg Leu Ala Arg His
    290                 295                 300

Ser Leu Gly Leu Arg Ser Leu Gly Leu Thr Met Arg Arg Cys Ala Arg
305                 310                 315                 320

Glu Phe Gly Leu Leu Leu Leu Phe Leu Cys Val Ala Met Ala Leu Phe
                325                 330                 335

Ala Pro Leu Val His Leu Ala Glu Arg Glu Leu Gly Ala Arg Arg Asp
            340                 345                 350

Phe Ser Ser Val Pro Ala Ser Tyr Trp Trp Ala Val Ile Ser Met Thr
        355                 360                 365

Thr Val Gly Tyr Gly Asp Met Val Pro Arg Ser Leu Pro Gly Gln Val
    370                 375                 380

Val Ala Leu Ser Ser Ile Leu Ser Gly Ile Leu Leu Met Ala Phe Pro
385                 390                 395                 400

Val Thr Ser Ile Phe His Thr Phe Ser Arg Ser Tyr Ser Glu Leu Lys
                405                 410                 415

Glu Gln Gln Gln Arg Ala Ala Ser Pro Glu Pro Ala Leu Gln Glu Asp
            420                 425                 430

Ser Thr His Ser Ala Thr Ala Thr Glu Asp Ser Ser Gln Gly Pro Asp
        435                 440                 445

Ser Ala Gly Leu Ala Asp Asp Ser Ala Asp Ala Leu Trp Val Arg Ala
    450                 455                 460

Gly Arg
465

<210> 3
<211> 1446
<212> DNA
<213> Maus

<220>
<221> CDS
<222> (1)..(1446)
<223> reifes murines Kv6.2 Protein

<400> 3
atg gcc cgg ctc ctg ggg cac ccg gag gcc ccc gac gcg gaa cct ggc      48
Met Ala Arg Leu Leu Gly His Pro Glu Ala Pro Asp Ala Glu Pro Gly
  1               5                  10                  15

agc gca ggc cga cag ggc cgt ggc ggc cgc ggg gcc cgg gcg cgc cac      96
Ser Ala Gly Arg Gln Gly Arg Gly Gly Arg Gly Ala Arg Ala Arg His
             20                  25                  30

gtc gtt atc aac atc tgg ggc tgc agg gtg cgt ctg gcc tgg gcc gcg    144
Val Val Ile Asn Ile Trp Gly Cys Arg Val Arg Leu Ala Trp Ala Ala
        35                  40                  45

ctg gcc cgc tgt ctc ctg gcg cgc ctc gag cgc ctg cga acc tgc cgc    192
Leu Ala Arg Cys Leu Leu Ala Arg Leu Glu Arg Leu Arg Thr Cys Arg
    50                  55                  60

ggc cac gag aaa ctg ctg cgc gtg tgt tac gac tac gac atg agc cgc    240
Gly His Glu Lys Leu Leu Arg Val Cys Tyr Asp Tyr Asp Met Ser Arg
65                  70                  75                  80

gac aaa ttc ttc ttc gaa ggc agc ccg tgc gct ttc ggc ccc atc gtg    288
Asp Lys Phe Phe Phe Glu Gly Ser Pro Cys Ala Phe Gly Pro Ile Val
                85                  90                  95

gcg ctg ctg cgc gcc cgc aaa gtg agg gtg ctg cgc ggc cct tgc gcc    336
Ala Leu Leu Arg Ala Arg Lys Val Arg Val Leu Arg Gly Pro Cys Ala
            100                 105                 110

ctg gcc ttc cgc gaa aaa gtg gcc tac tgg ggc atc gac gaa acg cgg    384
Leu Ala Phe Arg Glu Lys Val Ala Tyr Trp Gly Ile Asp Glu Thr Arg
        115                 120                 125

ctg gaa cgc tgc tgc ctg cgc cgc ctg cgc cgc cgc gag gag gag gcc    432
Leu Glu Arg Cys Cys Leu Arg Arg Leu Arg Arg Arg Glu Glu Glu Ala
    130                 135                 140

ccc gag gcc agc gcc gcg cag ccc gcc cga ggg ccg cag acc acc ccc    480
Pro Glu Ala Ser Ala Ala Gln Pro Ala Arg Gly Pro Gln Thr Thr Pro
145                 150                 155                 160

cgc cga gcc ctg gga ccc agc ggg cgg ctg gag aga ggc aga cgg cgc    528
Arg Arg Ala Leu Gly Pro Ser Gly Arg Leu Glu Arg Gly Arg Arg Arg
                165                 170                 175

ttg cga gac gtg gtg gag aac ccg cac tcc ggg ctg gcg ggc atc ttt    576
Leu Arg Asp Val Val Glu Asn Pro His Ser Gly Leu Ala Gly Ile Phe
            180                 185                 190

ttc gca tat gtc tcc gtg gct ttc gtg gcc gtc aca gcc gtc ggc ttg    624
Phe Ala Tyr Val Ser Val Ala Phe Val Ala Val Thr Ala Val Gly Leu
        195                 200                 205

tgc ctg agc acc atg ccg gat gtc cgc gca gaa gag gaa cgg ggc gag    672
Cys Leu Ser Thr Met Pro Asp Val Arg Ala Glu Glu Glu Arg Gly Glu
    210                 215                 220

tgc tcc aca aag tgc cgc aac ctg ttc gtg ctg gag acg gtg tgc gtg    720
Cys Ser Thr Lys Cys Arg Asn Leu Phe Val Leu Glu Thr Val Cys Val
225                 230                 235                 240

gcc tgg ttc tcc ttc gag ttc ctg ctg cgc tcc ctg cag gct gag agc    768
Ala Trp Phe Ser Phe Glu Phe Leu Leu Arg Ser Leu Gln Ala Glu Ser
                245                 250                 255

aag tgc gcc ttc ctc cgg acg ccg ctt gcc atc atc gac atc ctg gcc    816
Lys Cys Ala Phe Leu Arg Thr Pro Leu Ala Ile Ile Asp Ile Leu Ala
            260                 265                 270

atc ctg ccc tta tac gtg tcg ctg ctc gcg gga ctg gcg gca ggg ccc    864
Ile Leu Pro Leu Tyr Val Ser Leu Leu Ala Gly Leu Ala Ala Gly Pro
        275                 280                 285

acg ggc agc aag atg ctg gag cgc gcg ggt ctg gtg ctg cgg ctg ctg    912
Thr Gly Ser Lys Met Leu Glu Arg Ala Gly Leu Val Leu Arg Leu Leu

290                 295                 300

cgg gcg ctg cgc gtg ctc tac gtg atg cgc ctg gcg cgc cac tcg ttg     960
Arg Ala Leu Arg Val Leu Tyr Val Met Arg Leu Ala Arg His Ser Leu
305                 310                 315                 320

ggg ctg cgc tcg ctt ggc ctc acc gtg cgc cgc tgc gcg cgc gag ttc     1008
Gly Leu Arg Ser Leu Gly Leu Thr Val Arg Arg Cys Ala Arg Glu Phe
                325                 330                 335

gga ctg ctg ctg ctc ttc ctc tgc gtg gcc atg gcg ctc ttc gcg ccg     1056
Gly Leu Leu Leu Leu Phe Leu Cys Val Ala Met Ala Leu Phe Ala Pro
            340                 345                 350

ctc gtg cac ctg gct gag cgc gag ctg ggc gct cac cgc gac ttc tcc     1104
Leu Val His Leu Ala Glu Arg Glu Leu Gly Ala His Arg Asp Phe Ser
        355                 360                 365

agc gtc ccc gcc agc tac tgg tgg gca gtc atc tcc atg acc acc gtg     1152
Ser Val Pro Ala Ser Tyr Trp Trp Ala Val Ile Ser Met Thr Thr Val
    370                 375                 380

ggc tat gga gac atg gtg ccg cgc agc ctt ccg ggc cag gtg gtg gcg     1200
Gly Tyr Gly Asp Met Val Pro Arg Ser Leu Pro Gly Gln Val Val Ala
385                 390                 395                 400

ctg agc agc atc ctc agc ggc atc ctg ctc atg gcc ttc cct gtc acc     1248
Leu Ser Ser Ile Leu Ser Gly Ile Leu Leu Met Ala Phe Pro Val Thr
                405                 410                 415

tcc atc ttc cac acc ttc tcg cgc tcc tat tcg gag ctc aag gag cag     1296
Ser Ile Phe His Thr Phe Ser Arg Ser Tyr Ser Glu Leu Lys Glu Gln
            420                 425                 430

caa cag cgc gcg gcc agc cct gaa ccg gcc ctg cgc gag gac agc acg     1344
Gln Gln Arg Ala Ala Ser Pro Glu Pro Ala Leu Arg Glu Asp Ser Thr
        435                 440                 445

cgt gat gac agt aca cgt tcg gcc agc gcc act gag gac agc tct cag     1392
Arg Asp Asp Ser Thr Arg Ser Ala Ser Ala Thr Glu Asp Ser Ser Gln
    450                 455                 460

gac cct gag acc gca ggc gcg gca ggg aac ttg ccg ggc cgg gtg gga     1440
Asp Pro Glu Thr Ala Gly Ala Ala Gly Asn Leu Pro Gly Arg Val Gly
465                 470                 475                 480

ccc tga                                                             1446
Pro

<210> 4
<211> 481
<212> PRT
<213> Maus

<400> 4
Met Ala Arg Leu Leu Gly His Pro Glu Ala Pro Asp Ala Glu Pro Gly
1               5                   10                  15

Ser Ala Gly Arg Gln Gly Arg Gly Gly Arg Gly Ala Arg Ala Arg His
            20                  25                  30

Val Val Ile Asn Ile Trp Gly Cys Arg Val Arg Leu Ala Trp Ala Ala
        35                  40                  45

Leu Ala Arg Cys Leu Leu Ala Arg Leu Glu Arg Leu Arg Thr Cys Arg
    50                  55                  60

Gly His Glu Lys Leu Leu Arg Val Cys Tyr Asp Tyr Asp Met Ser Arg
65                  70                  75                  80

Asp Lys Phe Phe Phe Glu Gly Ser Pro Cys Ala Phe Gly Pro Ile Val
                85                  90                  95

Ala Leu Leu Arg Ala Arg Lys Val Arg Val Leu Arg Gly Pro Cys Ala
            100                 105                 110

Leu Ala Phe Arg Glu Lys Val Ala Tyr Trp Gly Ile Asp Glu Thr Arg
        115                 120                 125

Leu Glu Arg Cys Cys Leu Arg Arg Leu Arg Arg Arg Glu Glu Glu Ala
    130                 135                 140

Pro Glu Ala Ser Ala Ala Gln Pro Ala Arg Gly Pro Gln Thr Thr Pro
145                 150                 155                 160

Arg Arg Ala Leu Gly Pro Ser Gly Arg Leu Glu Arg Gly Arg Arg Arg
                165                 170                 175

Leu Arg Asp Val Val Glu Asn Pro His Ser Gly Leu Ala Gly Ile Phe
            180                 185                 190

Phe Ala Tyr Val Ser Val Ala Phe Val Ala Val Thr Ala Val Gly Leu
        195                 200                 205

Cys Leu Ser Thr Met Pro Asp Val Arg Ala Glu Glu Glu Arg Gly Glu
    210                 215                 220

Cys Ser Thr Lys Cys Arg Asn Leu Phe Val Leu Glu Thr Val Cys Val
225                 230                 235                 240

Ala Trp Phe Ser Phe Glu Phe Leu Leu Arg Ser Leu Gln Ala Glu Ser
                245                 250                 255

Lys Cys Ala Phe Leu Arg Thr Pro Leu Ala Ile Ile Asp Ile Leu Ala
            260                 265                 270

Ile Leu Pro Leu Tyr Val Ser Leu Leu Ala Gly Leu Ala Ala Gly Pro
        275                 280                 285

Thr Gly Ser Lys Met Leu Glu Arg Ala Gly Leu Val Leu Arg Leu Leu
    290                 295                 300

Arg Ala Leu Arg Val Leu Tyr Val Met Arg Leu Ala Arg His Ser Leu
305                 310                 315                 320

Gly Leu Arg Ser Leu Gly Leu Thr Val Arg Arg Cys Ala Arg Glu Phe
                325                 330                 335

Gly Leu Leu Leu Leu Phe Leu Cys Val Ala Met Ala Leu Phe Ala Pro
            340                 345                 350

Leu Val His Leu Ala Glu Arg Glu Leu Gly Ala His Arg Asp Phe Ser
        355                 360                 365

Ser Val Pro Ala Ser Tyr Trp Trp Ala Val Ile Ser Met Thr Thr Val
    370                 375                 380

Gly Tyr Gly Asp Met Val Pro Arg Ser Leu Pro Gly Gln Val Val Ala
385                 390                 395                 400

Leu Ser Ser Ile Leu Ser Gly Ile Leu Leu Met Ala Phe Pro Val Thr
                405                 410                 415

Ser Ile Phe His Thr Phe Ser Arg Ser Tyr Ser Glu Leu Lys Glu Gln
            420                 425                 430

Gln Gln Arg Ala Ala Ser Pro Glu Pro Ala Leu Arg Glu Asp Ser Thr
        435                 440                 445

Arg Asp Asp Ser Thr Arg Ser Ala Ser Ala Thr Glu Asp Ser Ser Gln
    450                 455                 460

Asp Pro Glu Thr Ala Gly Ala Ala Gly Asn Leu Pro Gly Arg Val Gly
465                 470                 475                 480

Pro

<210> 5
<211> 620
<212> DNA
<213> Homo sapiens

<220>
<221> CDS
<222> (150)..(620)
<223> kodierender Bereich des humanen genomischen
      SacI/PstI-Fragmentes

<400> 5
gagctcacct ccgagggttc ggtgccggcc cggcccctgg atccccgcgg gcggacgcgc 60

tcccccagct cagccctcgc gaccctaacg cggtccgttc cttttgcagg agccgggcag 120

gagcccctcg gtccggtccg gccctgcgc atg gag cca tgg ccc tgc tcc ccg   173
                                Met Glu Pro Trp Pro Cys Ser Pro
                                1               5

ggc ggc ggc ggc ggg acc cgc gcc cgg cac gtc atc atc aac gtg ggc   221
Gly Gly Gly Gly Gly Thr Arg Ala Arg His Val Ile Ile Asn Val Gly
    10                  15                  20

ggc tgc cgc gtg cgc ctg gca tgg gcc gcg ctg gcg cga tgc ccc ctc   269
Gly Cys Arg Val Arg Leu Ala Trp Ala Ala Leu Ala Arg Cys Pro Leu
25                  30                  35                  40

gcg cgc ctg gag cgc ctg cgc gcc tgc cgc ggc cac gac gac ctg ctg   317
Ala Arg Leu Glu Arg Leu Arg Ala Cys Arg Gly His Asp Asp Leu Leu
                45                  50                  55

cgc gtg tgt gac gac tac gac gtg agc cgc gac gag ttc ttc ttc gac   365
Arg Val Cys Asp Asp Tyr Asp Val Ser Arg Asp Glu Phe Phe Phe Asp
            60                  65                  70

cgc agc ccg tgc gcc ttc cgc gcc atc gtg gcg ctt ttg cgc gca ggg   413
Arg Ser Pro Cys Ala Phe Arg Ala Ile Val Ala Leu Leu Arg Ala Gly
        75                  80                  85

aag ctg cga ctg ctg cgg ggc ccg tgc gcg ctg gcc ttc cga gac gag   461
Lys Leu Arg Leu Leu Arg Gly Pro Cys Ala Leu Ala Phe Arg Asp Glu
    90                  95                  100

ctg gcc tac tgg ggc atc gac gag gcg cgc atg gac tgc cgc tgc ctg   509
Leu Ala Tyr Trp Gly Ile Asp Glu Ala Arg Met Asp Cys Arg Cys Leu
105                 110                 115                 120

cgc cgc atg cgc cgc cgc gag gag gag gcg gcc gag gcc cgc gcg ggg   557
Arg Arg Met Arg Arg Arg Glu Glu Glu Ala Ala Glu Ala Arg Ala Gly
                125                 130                 135

ccg acg gag cgc ggg gcg cag ggg agc ccg gcg cgc gcc ctg gga cct     605
Pro Thr Glu Arg Gly Ala Gln Gly Ser Pro Ala Arg Ala Leu Gly Pro
            140                 145                 150

cgg ggg cgg ctg cag                                                 620
Arg Gly Arg Leu Gln
        155


<210> 6
<211> 157
<212> PRT
<213> Homo sapiens

<400> 6
Met Glu Pro Trp Pro Cys Ser Pro Gly Gly Gly Gly Gly Thr Arg Ala
  1               5                  10                  15

Arg His Val Ile Ile Asn Val Gly Gly Cys Arg Val Arg Leu Ala Trp
             20                  25                  30

Ala Ala Leu Ala Arg Cys Pro Leu Ala Arg Leu Glu Arg Leu Arg Ala
         35                  40                  45

Cys Arg Gly His Asp Asp Leu Leu Arg Val Cys Asp Asp Tyr Asp Val
     50                  55                  60

Ser Arg Asp Glu Phe Phe Phe Asp Arg Ser Pro Cys Ala Phe Arg Ala
 65                  70                  75                  80

Ile Val Ala Leu Leu Arg Ala Gly Lys Leu Arg Leu Leu Arg Gly Pro
                 85                  90                  95

Cys Ala Leu Ala Phe Arg Asp Glu Leu Ala Tyr Trp Gly Ile Asp Glu
            100                 105                 110

Ala Arg Met Asp Cys Arg Cys Leu Arg Arg Met Arg Arg Arg Glu Glu
        115                 120                 125

Glu Ala Ala Glu Ala Arg Ala Gly Pro Thr Glu Arg Gly Ala Gln Gly
    130                 135                 140

Ser Pro Ala Arg Ala Leu Gly Pro Arg Gly Arg Leu Gln
145                 150                 155


<210> 7
<211> 392
<212> DNA
<213> Maus

<220>
<221> CDS
<222> (1)..(162)
<223> Maus Kv6.2 (3'-Bereich)

<400> 7
gag ctc aag gag cag caa cag cgc gcg gcc agc cct gaa ccg gcc ctg      48
Glu Leu Lys Glu Gln Gln Gln Arg Ala Ala Ser Pro Glu Pro Ala Leu
  1               5                  10                  15

cgc gag gac agc acg cgt gat gac agt aca cgt tcg gcc agc gcc act      96
Arg Glu Asp Ser Thr Arg Asp Asp Ser Thr Arg Ser Ala Ser Ala Thr
             20                  25                  30

gag gac agc tct cag gac cct gag acc gca ggc gcg gca ggg aac ttg 144
Glu Asp Ser Ser Gln Asp Pro Glu Thr Ala Gly Ala Ala Gly Asn Leu
        35                  40                  45

ccg ggc cgg gtg gga ccc tgagctgtac tgagaacttc aagagagtca 192
Pro Gly Arg Val Gly Pro
    50

agagcctcgg aggacaccta gcgccaactg acccaggagt tggcaaactt ggtctggcat 252

atccttgcag ctggctcgcc tccccaagca attcccgagc ttcgcatagc ctggaggata 312

tagcaacctg gctttctttt tgcttttatt ttcccttcag ttttaaattt tctatggcta 372

attaaaaata attgagtccc 392

<210> 8
<211> 54
<212> PRT
<213> Maus

<400> 8
Glu Leu Lys Glu Gln Gln Gln Arg Ala Ala Ser Pro Glu Pro Ala Leu
1               5                   10                  15

Arg Glu Asp Ser Thr Arg Asp Asp Ser Thr Arg Ser Ala Ser Ala Thr
            20                  25                  30

Glu Asp Ser Ser Gln Asp Pro Glu Thr Ala Gly Ala Ala Gly Asn Leu
        35                  40                  45

Pro Gly Arg Val Gly Pro
    50

<210> 9
<211> 22
<212> RNA
<213> Homo sapiens

<220>
<221> misc_RNA
<222> (1)..(22)
<223> Intron/Exon-Übergang

<220>
<221> CDS
<222> (1)..(15)
<223> kodierender Bereich/Exon

<400> 9
gcc gag gag gag cgg gtgagcg 22
Ala Glu Glu Glu Arg
1               5

<210> 10
<211> 5
<212> PRT
<213> Homo sapiens

<400> 10
Ala Glu Glu Glu Arg
1               5

<210> 11
<211> 36
<212> RNA
<213> Homo sapiens

<220>
<221> misc_RNA
<222> (1)..(36)
<223> Intron/Exon-Übergang

<220>
<221> CDS
<222> (25)..(36)
<223> kodierender Bereich/Exon

<400> 11
ccccgtgtcc cctctccccc gcag ggc gag tgc tcc 36
Gly Glu Cys Ser
1

<210> 12
<211> 4
<212> PRT
<213> Homo sapiens

<400> 12
Gly Glu Cys Ser
1

<210> 13
<211> 27
<212> RNA
<213> Maus

<220>
<221> CDS
<222> (1)..(27)
<223> kodierender Bereich: Übergang Exon 1 zu Exon 2

<400> 13
gca gaa gag gaa cgg ggc gag tgc tcc 27
Ala Glu Glu Glu Arg Gly Glu Cys Ser
1               5

<210> 14
<211> 9
<212> PRT
<213> Maus

<400> 14
Ala Glu Glu Glu Arg Gly Glu Cys Ser
1               5

<210> 15
<211> 27
<212> RNA
<213> Homo sapiens

<220>
<221> CDS
<222> (1)..(27)
<223> kodierender Bereich: Übergang Exon 1 zu Exon 2

<400> 15
gcc gag gag gag cgg ggc gag tgc tcc                                    27
Ala Glu Glu Glu Arg Gly Glu Cys Ser
 1               5

<210> 16
<211> 9
<212> PRT
<213> Homo sapiens

<400> 16
Ala Glu Glu Glu Arg Gly Glu Cys Ser
 1               5

<210> 17
<211> 32
<212> DNA
<213> Künstliche Sequenz

<220>
<223> Beschreibung der künstlichen Sequenz: SEAM 1;
      Primer für PCR

<220>
<221> CDS
<222> (18)..(32)
<223> Kodierender Bereich im Primer

<400> 17
agttactctt caccacc atg gag cca tgg ccc                                 32
                   Met Glu Pro Trp Pro
                    1               5

<210> 18
<211> 5
<212> PRT
<213> Künstliche Sequenz

<400> 18
Met Glu Pro Trp Pro
 1               5

<210> 19
<211> 32
<212> DNA
<213> Künstliche Sequenz

<220>
<223> Beschreibung der künstlichen Sequenz: SEAM 6;
      Primer für PCR

<400> 19
agttactctt caccccgctc ctcctcggcg cg                                    32

<210> 20
<211> 30
<212> DNA
<213> Künstliche Sequenz

<220>
<223> Beschreibung der künstlichen Sequenz: SEAM 5;
      Primer für PCR

<220>
<221> CDS
<222> (13)..(30)
<223> Kodierender Bereich im Primer

<400> 20
agttactctt ca ggg cga gtg ctc ccc caa                                30
              Gly Glu Cys Ser Pro Lys
                1               5


<210> 21
<211> 6
<212> PRT
<213> Künstliche Sequenz

<400> 21
Gly Glu Cys Ser Pro Lys
  1               5


<210> 22
<211> 27
<212> DNA
<213> Künstliche Sequenz

<220>
<223> Beschreibung der künstlichen Sequenz: SEAM 4;
      Primer für PCR

<400> 22
agttactctt catgtgggcg gcgcagg                                        27


<210> 23
<211> 17
<212> DNA
<213> Künstliche Sequenz

<220>
<223> Beschreibung der künstlichen Sequenz: M13; Primer
      zum Sequenzieren

<400> 23
gtaaaacgac ggccagt                                                   17


<210> 24
<211> 19
<212> DNA
<213> Künstliche Sequenz

<220>
<223> Beschreibung der künstlichen Sequenz: Reverse;
      Primer zum Sequenzieren

<400> 24
ggaaacagct atgaccatg                                                 19

<210> 25
<211> 20
<212> DNA
<213> Künstliche Sequenz

<220>
<223> Beschreibung der künstlichen Sequenz: T3; Primer
      zum Sequenzieren

<400> 25
aattaaccct cactaaaggg                                                    20


<210> 26
<211> 19
<212> DNA
<213> Künstliche Sequenz

<220>
<223> Beschreibung der künstlichen Sequenz: T7; Primer
      zum Sequenzieren

<400> 26
ggaaacagct atgaccatg                                                     19


<210> 27
<211> 5
<212> DNA
<213> Künstliche Sequenz

<220>
<223> Beschreibung der künstlichen Sequenz: Kozak; Zum
      Einbau in Seam 1

<400> 27
ccacc                                                                    5